(19) **Europäisches Patentamt**

**European Patent Office**

**Office européen des brevets**

(11) **EP 0 782 375 B1**

(12) **EUROPEAN PATENT SPECIFICATION**

(45) Date of publication and mention
of the grant of the patent:
**23.04.2003 Bulletin 2003/17**

(51) Int Cl.[7]: **H05G 1/60**, G21K 1/02,
A61B 6/02

(21) Application number: **96309554.2**

(22) Date of filing: **30.12.1996**

(54) **Apparatus and method for removing scatter from an x-ray image**

Vorrichtung und Verfahren zur Entfernung der Streuung aus Röntgenbildern

Appareil et procédé pour éliminer la dispersion d'une image par rayons x

(84) Designated Contracting States:
**DE FR GB**

(30) Priority: **29.12.1995 US 580602**
**03.10.1996 US 725375**

(43) Date of publication of application:
**02.07.1997 Bulletin 1997/27**

(73) Proprietor: **Advanced Optical Technologies, Inc.**
**East Hartford, Connecticut 06118 (US)**

(72) Inventor: **Chao, Yong-Sheng**
**Storrs, Connecticut 06268 (US)**

(74) Representative:
**Vaughan, Christopher Tammo et al**
**Haseltine Lake & Co.,**
**Imperial House,**
**15-19 Kingsway**
**London WC2B 6UD (GB)**

(56) References cited:
**EP-A- 0 105 618       EP-A- 0 184 247**
**EP-A- 0 218 923       US-A- 4 389 729**
**US-A- 4 829 552       US-A- 5 440 647**

• **MEDICAL PHYSICS, vol. 17, no. 5, September 1990, NEW YORK US, pages 866-875, XP000170780 B.K. STEWART ET AL.: "SINGLE-EXPOSURE DUAL-ENERGY COMPUTED RADIOGRAPHY"**
• **JOURNAL OF APPLIED PHOTOGRAPHIC ENGINEERING, vol. 9, no. 6, December 1983, SPRINGFIELD US, pages 184-195, XP002027697 M.J. YAFFE ET AL.: "SCATTERED RADIATION IN DIAGNOSTIC RADIOLOGY: MAGNITUDES, EFFECTS, AND METHODS OF REDUCTION"**

**Description**

This invention relates generally to digital x-ray imaging and, more particularly, relates to methods and apparatuses for reducing scatter in two-dimensional x-ray imaging and two-dimensional dual-energy x-ray imaging.

Recent advances in semiconductor industry have resulted in the ability of fabricating large-format two-dimensional integrated detector arrays for x-ray detection. These arrays have on the order of one million detector cells and provide instant acquisition of two-dimensional x-ray images with exceedingly high quality.

Scatter, which results from those x-rays that strike objects and deflect in random directions, has been a difficult and on-going problem in x-ray imaging using two-dimensional detectors. For example, in projection chest radiography, scatter typically accounts for between approximately 30% and 50% of the total amount of x-rays detected.

Two-dimensional detectors are exposed to wide-angle random scatter. Scatter tends to degrade image quality, and to block the way for quantitative imaging. For example, with scatter present, three-dimensional image reconstruction cannot be implemented.

Embodiments of the present invention use the dual-energy x-ray imaging method as a foundation for the method of eliminating scatter. However, the dual-energy x-ray imaging methods of prior art are not suitable for this purpose.

All the dual-energy x-ray data decomposition methods of the prior art have a common approach: linearization.

It is a consensus that none of current linearization methods is suitable for use with million-pixel two-dimensional detector arrays. One object of the invention is to provide a substantially improved dual-energy x-ray imaging method suitable for two-dimensional detector arrays.

Currently, there are two basic methods for reducing scatter in two-dimensional x-ray imaging. The first method uses an anti-scatter grid (see MEDICAL PHYSICS, vol. 17, no. 5, September 1990, New York, US) or improved grid devices to slightly relieve scatter effects on images. By increasing the air gap between the subject and the detector, the scatter can also be reduced, but at the same time, the image is blurred due to the geometric distance the x-rays have to travel.

The second method to reduce scatter is to calculate theoretical estimates. Theoretical methods, including Monte Carlo simulation methods and analytical deconvolution methods, can only give crude predictions.

According to the present invention, there is provided a two-dimensional x-ray imaging system for taking images of a subject as defined in claim 1 and a method for taking a two-dimensional x-ray image of a subject as defined in claim 8.

The dual-energy x-ray data decomposition method according to an embodiment of the invention consists in directly solving the dual energy nonlinear equation system, it includes: (1) Constructing an explicit quantitative equation system $D_H = D_H(b,s)$ and $D_L = D_L(b,s)$ for each detector according to the nonlinear dual-energy x-ray imaging fundamental equation system in its original form. (2) reconstructing an equation system $b = b(D_H,D_L)$ and $s = s(D_H,D_L)$ by numerically inverting the equation system of step 1.(3) Determining the desired values for b and s from the available data pair $(D_H,D_L)$ by using the numerical equations of step 2, or determining the desired values for $D_H,D_L$, from the available data pair (b,s) by using the numerical equations of step 1. (4) Maintaining the accuracy at each step to be as high as real number analytical solutions can provide.

Embodiments of the present invention advantageously comprise an x-ray detection system that has the following features: (1) using a beam selection means to physically separates the primary x-rays from the scatter x-rays at a number of selected locations; (2) using a front detector assembly detects the primary and scatter x-rays, using a rear detector assembly detects only the primary x-rays at selected locations without scatter.

The method for eliminating scatter x-rays advantageously includes: (1) using a dual-energy method to determine the primary image of the front detector at the corresponding selected locations from the image data of the rear detector assembly; (2) using an interpolation method to extend the scatter component of the front detector image determined at a the selected locations to the entire image area of the front detector. Following this, a complete separation of scatter and primary images for the front detector at high spatial resolution is achieved.

Thus, embodiments of the present invention seek to provide an apparatus and method for substantially eliminating the effects of scatter on two-dimensional x-ray detectors.

Embodiments of the present invention may also seek to provide an apparatus and a method for scatter-free dual-energy x-ray imaging using two-dimensional detectors and to provide two high accuracy material composition images of a subject at the spatial resolution as high as a two-dimensional detector array can provide.

For a better understanding of the present invention, and to show how it may be carried into effect, reference shall now be made by way of example to the accompany drawings, in which:

Fig. 1 is a diagram of the basic hardware of an embodiment of the present invention;
Fig. 2 is a curve describing a typical x-ray source energy spectrum used in embodiments of the present invention;
Fig. 3 is a flow diagram of the basic dual-energy data decomposition method using the hardware of Fig. 1;

Fig. 4 is a flow diagram of the basic method to eliminate scatter using the hardware of Fig. 1;
Fig. 5 is a diagram of a first embodiment of the present invention;
Fig. 6 is a flow diagram of the method of the first embodiment using the hardware of Fig. 5;
Figs. 7a and 7b are diagrams of a special case of the first embodiment of the present invention;
Fig. 8 is a flow diagram of the method of the special case using the hardware of Figs. 7a and 7b;
Fig. 9 is a diagram of a second embodiment of the present invention;
Fig. 10 is a flow diagram of the method of the second embodiment using the hardware of Fig. 9;

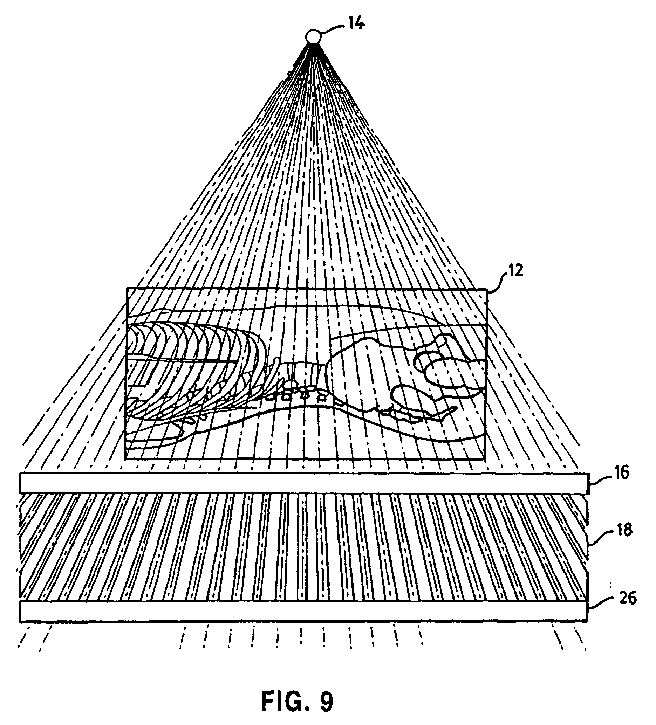

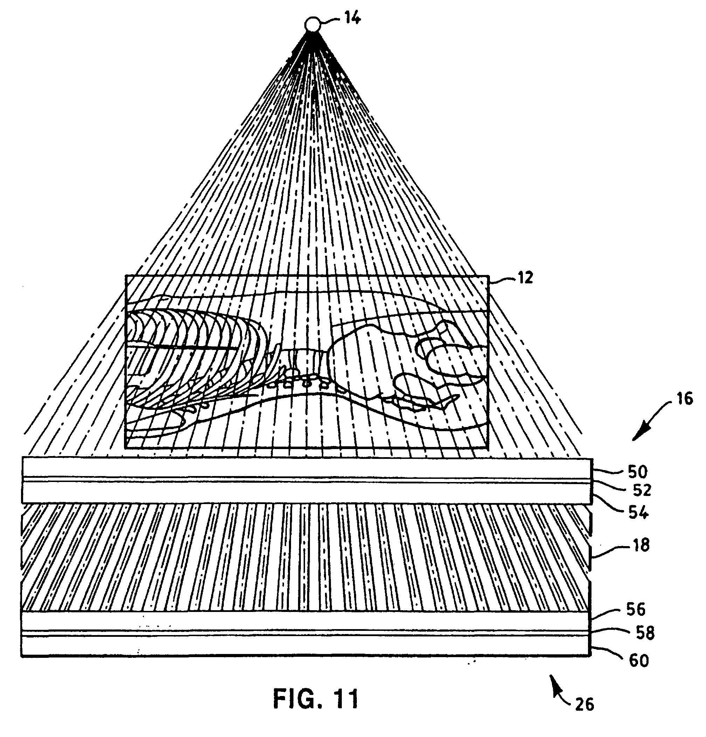

Fig. 11 is a diagram of a third embodiment of the present invention;
Fig. 12 is a flow diagram of the method of the third embodiment using the hardware of Fig. 11; and
Figs. 13a to 13d is a graphically representation of a method for inverting the nonlinear dual-energy equation systems.

Introduction

The basic apparatus includes five components: (1) an x-ray source, (2) a front two-dimensional x-ray detector assembly, (3) a beam selector, (4) a rear two-dimensional x-ray detector assembly, and (5) a computer.

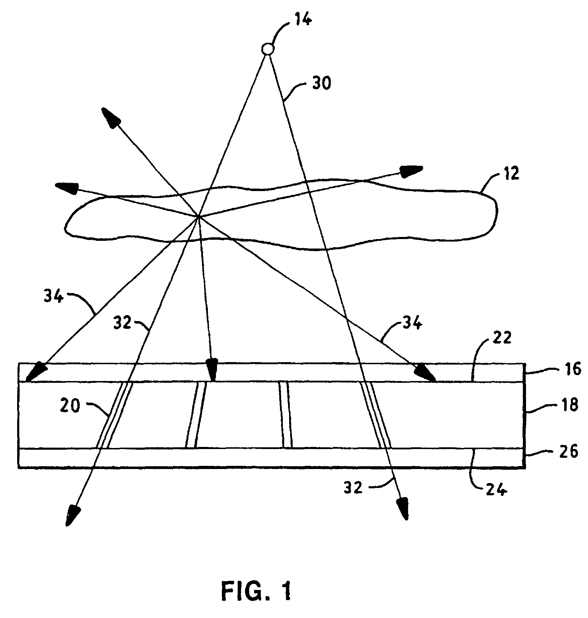

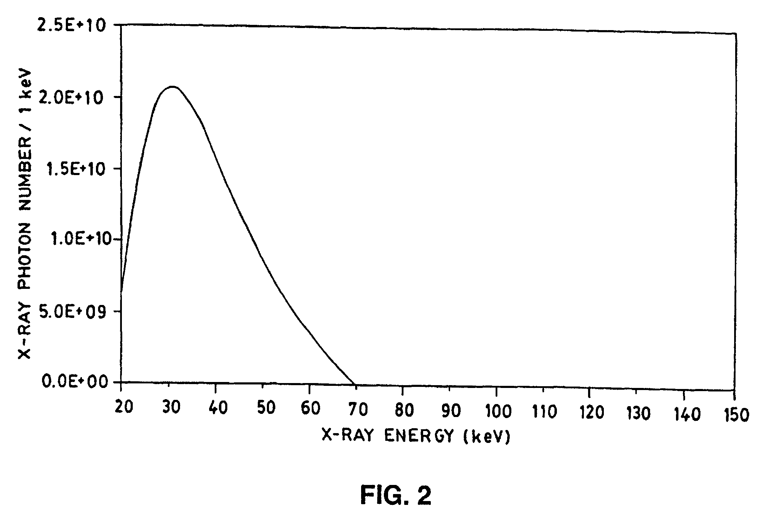

As shown in Fig. 1, the subject under examination 12 is located between the x-ray source 14 and the front detector assembly 16. The x-ray source 14 emits x-rays 30, preferably with an energy in the range of from 10 keV to 500 keV, as shown in Fig. 2. A portion of the x-rays 32 passes through the subject 12 directly to the front detector assembly 16 without a change in their direction of propagation. These x-rays 32 are called the primary x-rays and convey true information about the subject 12. The remainder of the x-rays 34 are randomly scattered as a result of interaction with the material of the subject 12. These x-rays 34 are called scatter and cause a distortion of the true information.

The front detector in the front detector assembly 16 has a number of detector types. The first uses thin film amorphous silicon as photodetection medium. An scintillation screen, is placed in close contact with the entire photosensitive area of the photodetector array. Preferably, the detector array has dimensions of 20 centimeters (cm) by 20 cm or 40 cm by 40 cm for a single detector module. The cell size for this detector array is in the range of from approximately 50 $\mu$m by 50 $\mu$m to approximately 1 mm by 1 mm.

A second type of detector array uses an amorphous selenium film or selenium alloy film as the x-ray sensitive medium. Other typical two-dimensional detector arrays include but not limited to, charge-couple device (CCD) detectors, thin-film thallium-bromide-based detector arrays, avalanche silicon detector arrays, and phosphor-stimulatable computed radiography screens.

The front detector assembly 16 conveys an image that is a combination of the primary x-rays 32 and the scatter 34, and is denoted by

$$D_{fh}(x,y) = D_{fPh}(x,y) + D_{fSh}(x,y) \tag{1}$$

where $D_f$ denotes an image in the front detector assembly 16 and (x,y) denotes the two-dimensional Cartesian coordinates of a cell of the front detector assembly 16. $D_{fPh}(x,y)$ denotes the contribution from the primary x-rays 32 and $D_{fSh}(x,y)$ denotes the contribution from the scatter 34.

The beam selector is sandwiched between the front detector assembly 16 and the rear detector assembly 26. A preferred embodiment of the x-ray beam selector 18 is a quantity of x-ray-absorbent material having a large number of holes 20. The holes 20 are fabricated such that their axes are aligned with the travel direction of the primary x-rays 32. As a result, the holes 20 permit all x-rays traveling along the axes of the holes 20 to pass through, while all x-rays traveling in directions deviating slightly from the hole axes are completely absorbed by the bulk material of the beam selector 18. Because the holes 20 always have a finite size, a small portion of randomly scattered x-rays from the image subject 12 still can reach the rear detector assembly 26. However, as long as the hole size 20 is small and the thickness of the beam selector 18 is sufficiently large, this portion of the scatter 34 can be controlled to be negligibly small. Preferably, the holes 20 have a diameter that is in the range of approximately from 0.5 mm to 10 mm. Preferably, there are as many holes as possible in the beam selector 18. However, the beam selector material must occupy enough area so that the amount of scatter 34 is reduced to an insignificant quantity. A compromise based on these factors results in a pitch that is preferably between 2 mm and 50 mm. Preferably, the x-ray source 14 is located between 20 cm and 150 cm from the rear surface 24 of the beam selector 18. The invention holds equally true when the x-ray source is a point source or has a finite size.

The term "selected location" is defined as the location on the rear detector assembly 28, or on a rear detector, where, due to the function of the beam selector, only primary x-rays are received, and the scatter x-rays are substantially blocked. The "selected projection line" is defined as a straight line connecting the x-ray source to a point in the "selected location". The rear detector cells at the selected locations have a fixed geometric relation with some of the front detector cells. This relation is established by drawing a selected projection line from the x-ray source 14 through the beam selector 18 to the selected location. This selected projection line intersects the rear detector surface at a rear detector cell at a coordinate (i,j), and intersects the front detector surface at a front detector cell at a coordinate (x(i),y(j)). Here (x(i),y(j)) denote the Cartesian coordinate (x,y) of the front detector cell in the front detector assembly 16 closest to the selected projection line. The relationship between (i,j) and (x(i),y(j)) is experimentally established for all of the holes 20 of the beam selector 18 and stored in computer. The images represented by the composite of the signals from the detector cells of only one detector on the selected projection lines are low-resolution images and are represented by the subscript lower-case 1. The images represented by the composite of the signals from all the front detector cells are high-resolution images and are represented by the subscript lower-case h.

In connection with the material composition of the image subject, four quantities are defined. b(i,j) and s(i,j) are defined as the selected projection mass densities along the selected projection line (i,j). b(x,y) and s(x,y) are defined as the projection mass densities along the projection line (x,y). The "projection mass density" is defined as the integrated total mass of the image subject along the projection line per unit area. Because the projection mass density is not dependent on the size of detector cells, b(x(i),y(j)) = b(i,j) and s(x(i),y(j)) = s(i,j).

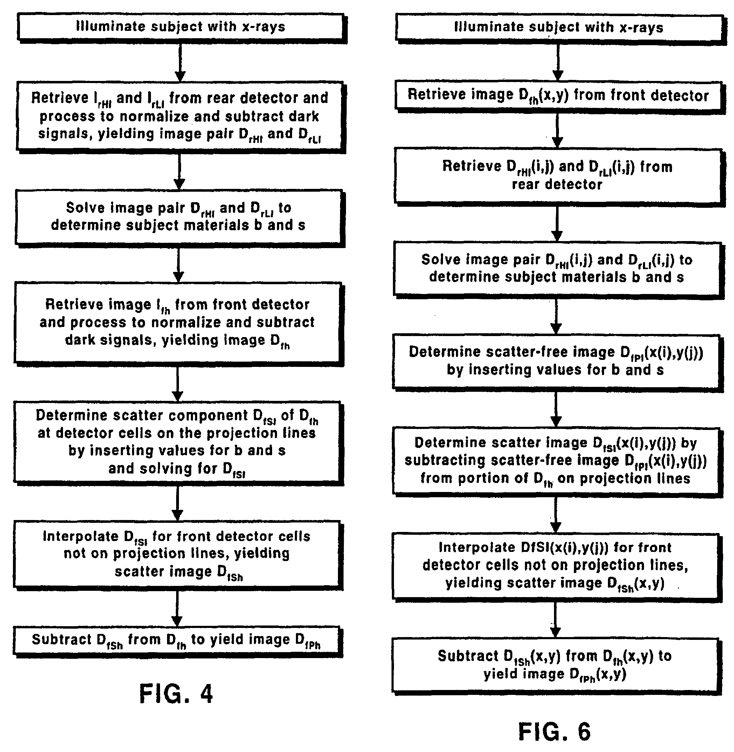

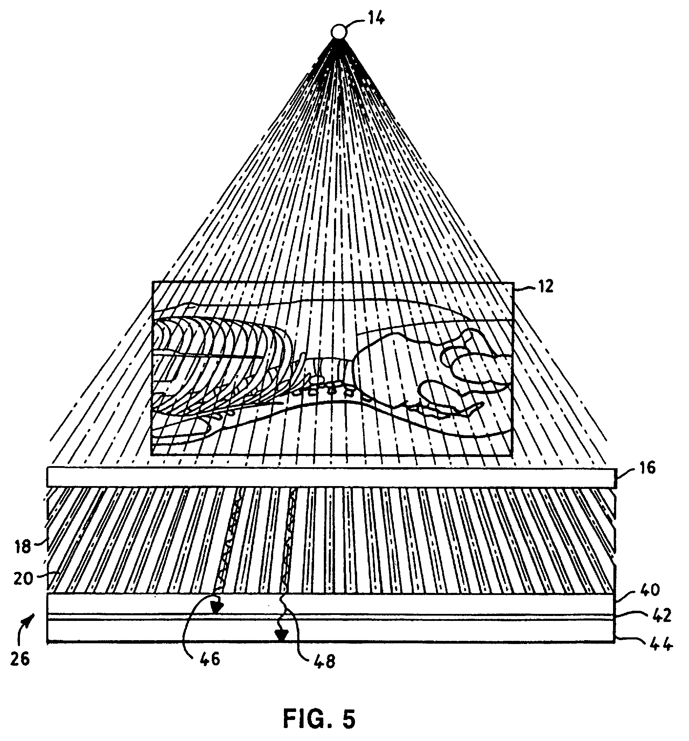

The method for eliminating scatter, shown in the flow diagram of Fig. 4, consists of the following steps: (1) illuminating the subject with x-rays from the x-ray source 14; (2) acquiring a low-resolution image pair $I_{rHl}(i,j)$ and $I_{rLl}(i,j)$ from the rear detector assembly and processing it to normalize and to subtract dark signals, yielding a low-resolution image pair $D_{rHl}(i,j)$ and $D_{rLl}(i,j)$ that are functions of the subject materials, where "acquiring an image" is defined as transferring, via electronic control circuits, the electrical signals induced by the x-ray illumination on each detector cell from a detector array to computer memory; (3) solving the image pair $D_{rHl}(i,j)$ and $D_{rLl}(i,j)$ to determine the selected projection mass density of the subject materials b(i,j) and s(i,j) according to dual energy data decomposition methods; (4) acquiring a high-resolution image $I_{fh}(x,y)$ from the front detector and processing it to normalize and to subtract dark signals, yielding a high-resolution image $D_{fh}(x,y)$, which is the sum of primary x-rays and scatter x-rays; (5) calculating the low-resolution primary image of the front detector $D_{fPl}(x(i),y(j))$ at the detector cells on the selected projection lines from the mass projection densities b(i,j) and s(i,j); (6) subtracting $D_{fPl}(x(i),y(j))$ from $D_{fh}(x(i),y(j))$ to calculate the low-resolution scatter component $D_{fSl}(x(i),y(j))$ of the image $D_{fh}(x(i),y(j))$ at the detector cells on selected projection lines; (7) interpolating $D_{fSl}(x(i),y(j))$ for those front detector cells not on the selected projection lines, yielding the high-resolution scatter image $D_{fSh}(x,y)$; and (8) subtracting the image $D_{fSh}(x,y)$ from $D_{fh}(x,y)$ to yield an image $D_{fPh}(x,y)$, which is a full two-dimensional image of the subject at the front detector after scatter x-rays have been substantially eliminated.

Because the data decomposition method of the present invention is rigorous and accurate and the mathematical calculations can be performed with good precision, as long as the experimental image data acquired from the detectors 16, 26 is highly accurate, the final result will be a highly accurate primary image of the subject 12.

First embodiment

In the first embodiment of the apparatus, shown in Fig. 5, the rear detector assembly 26 is constructed as a dual-energy x-ray imaging detector assembly. It has a low-energy two-dimensional detector 40, an x-ray energy spectral filter 42, and a high-energy two-dimensional detector 44. After the filter 42, x-ray energy is higher than that before the filter 42. The image detected by the low-energy detector 40 is denoted by $D_{rL}(i,j)$ and the image detected by the high-energy detector 44 is denoted by $D_{rH}(i,j)$. Preferably, the low-energy x-rays have an average energy of from 10 keV to 100 keV and high-energy x-rays have an average energy of from 30 keV to 500 keV, with the high-energy x-rays having a higher energy than the low-energy x-rays.

The purpose of measuring a pair of dual-energy primary images $D_{rH}(i,j)$ and $D_{rL}(i,j)$ of the rear detector assembly is to provide data for uniquely determining the corresponding primary low resolution image $D_{fpl}(x(i),y(j))$ at the front detector.

A flow diagram describing the method for determining a scatter-free image using the hardware of the first embodiment is shown in Fig. 6. The x-ray source 14 emits x-rays with a uniform angular distribution and with an energy spectrum of $\Phi_0(E)$. The x-rays passing through the subject 12 carry information on the thickness and material composition of the subject 12, expressed as projection mass density in units of grams/centimeters$^2$ (g/cm$^2$). The image induced by the x-rays incident on the front detector 16 is denoted as $D_{fh}(x,y)$ and is

$$D_{fh}(x,y) = \int [\Phi_0(E) \times \exp(-(\mu_b(E) \times b(x,y) +$$

$$\mu_s(E) \times s(x,y))] \times S_f(E)dE +$$

$$\int \Phi_s(E) \times S_f(E)dE \tag{2}$$

where $\mu_b(E)$ is the mass absorption coefficient of bone tissue and $\mu_s(E)$ is the mass absorption coefficient of soft tissue, with both $\mu_b(E)$ and $\mu_s(E)$ expressed in units of centimeter$^2$/gram (cm$^2$/g). Both of these values are known, having been determined experimentally and tabulated many years ago. $S_f(E)$ is the x-ray spectral sensitivity (the electrical signal amplitude from the detector as a function of the number of x-rays with energy E incident upon the detector) of the front detector 16. The term $\int \Phi_s(E) \times S_f(E)dE$ represents the signal caused by scatter.

The rear detector assembly 26 has two detectors 40, 44, so there are two low-resolution images $D_{rLl}(i,j)$ and $D_{rHl}(i,j)$, which are

$$D_{rLl}(i,j) = \int [\Phi_0(E) \times \exp(-(\mu_b(E) \times b(i,j) +$$

$$\mu_s(E) \times s(i,j))] \times S_{rL}(E)dE \tag{3a}$$

and

$$D_{rHl}(i,j) = \int [\Phi_0(E) \times \exp(-(\mu_b(E) \times b(i,j) +$$

$$\mu_s(E) \times s(i,j))] \times S_{rH}(E)dE \tag{3b}$$

Equations 3a and 3b constitute a simultaneous equation system, where the values for the signal pair $D_{rLl}(i,j)$, $D_{rHl}(i,j)$ are known quantities, being evaluated from the electrical signals of the rear detectors 40, 44. b(i,j) and s(i,j) are the unknown quantities for which equation pair 3a, 3b must be solved, as described below. Generally speaking, in mathematics, such a nonlinear simultaneous equation system may have an infinite number of solutions, may have multiple-value solutions, or may not have any solution. However, for the specific physical case of x-ray imaging, where the energy range is limited, preferably between 10 keV and 500 keV, it can be mathematically proved that a unique solution always exists for the nonlinear equation system 3a and 3b.

Accurate b(i,j) and s(i,j) are calculated by the data decomposition method of the present invention, as will be described below. Now that the values for b(i,j) and s(i,j) are known, the front low-resolution scatter-free image $D_{fPl}(x,y)$ can be obtained for those front detector cells (x(i),y(j)) that are on the selected projection lines. $D_{fPl}(x(i),y(j))$ is the signal induced by only primary x-rays at the detector cell (x(i),y(j)) on the front detector, and is

$$D_{fPl}(x(i),y(j)) = \int [\Phi_0(E) \times \exp(-(\mu_b(E) \times$$

$$b(i,j) + \mu_s(E) \times s(i,j))] \times$$

$$S_f(E)dE \tag{4}$$

where (x(i),y(j)) is the coordinate of the front detector cell (x,y) lying on the same selected projection line as the rear detector cell (i,j) and the energy dependent function $\Phi_0(E) \times S_f(E)$ is given in calibration, as will be described in the data decomposition section below.

Next, the low-resolution front scatter image $D_{fSl}(x(i),y(j))$ is determined by applying equation 3,

$$D_{fSl}(x(i), (j)) = D_{fl}(x(i),y(j)) - D_{fPl}(x(i),y(j))$$

Because of the scatter is largely caused by Compton scattering, which has a rather smooth angular distribution, the low-resolution scatter image $D_{fSl}(x(i),y(j))$ can be extended to the entire (x,y) plane through interpolation without losing accuracy, yielding the high-resolution scatter image $D_{fSh}(x,y)$. The high-resolution scatter image $D_{fSh}(x,y)$ is subtracted from the experimentally measured image $D_{fh}(x,y)$, yielding the high-resolution scatter-free signal $D_{fPh}(x,y)$.

It is now clear why it is crucial to acquire a pair of dual-energy x-ray images $D_{rL}(i,j)$, $D_{rH}(i,j)$ to determine the scatter. The primary signals at the front detector $D_{fPl}(x(i),y(j))$ can be uniquely determined only when the material composition image pair b(i,j) and s(i,j) is known (see equation 7). If only one image at the rear detector is used, it would always be found that the signal ratio between the front detector and the rear detector is dependent on the energy spectrum or dependent on the image subject. Consequently, the primary image on the front detector could not be determined.

It is also noted that, as a supplement to this general rule, there are two special cases where the relationship between the signals on the image plane (i,j) and the image plane (x(i),y(j)) can be degenerated or simplified to be proportional to each other and independent of the image subject. Thus, for correcting scatter, it is not necessary to use the dual-energy method for these two special cases.

The first special case occurs when the range of the x-ray energy spectral distribution is sufficiently narrow so that the x-rays can be approximated as having only a single energy or a well-defined average energy $E_0$. The signals on the front detector and the signals on the rear detector become a constant ,independent of image subject and can be predetermined before using the system for imaging operations.

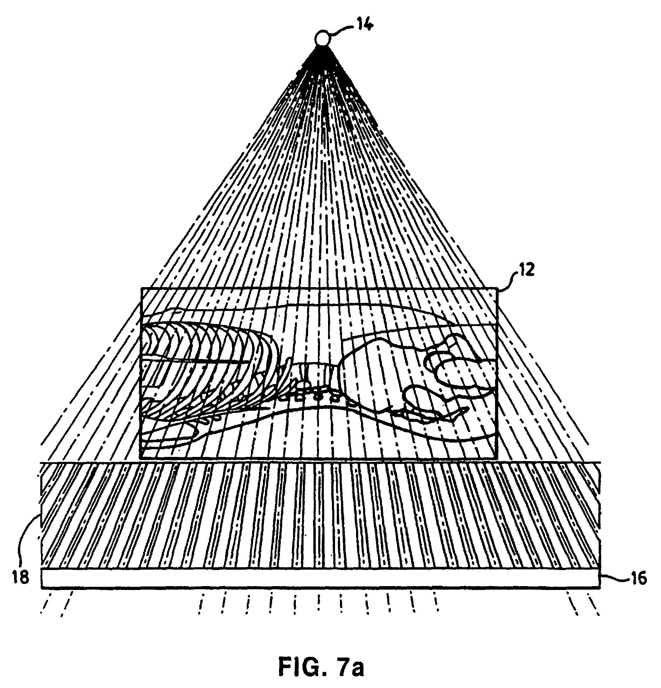

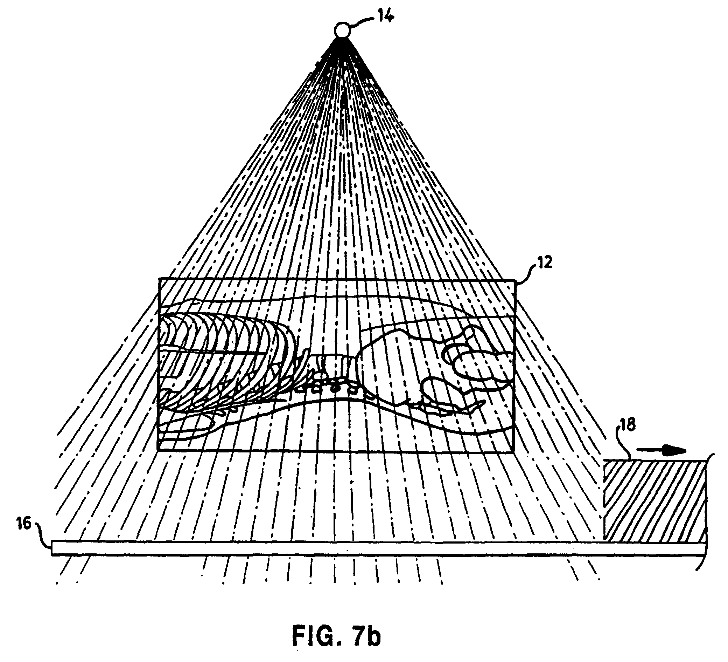

The second special case is shown in Figs. 7a and 7b, where only one detector is used for acquiring both the high resolution image $D_{fh}(x,y)$ and the low resolution image $D_{rl}(i,j)$. A mechanical device moves the beam selector to and away from the detector's front surface. When the beam selector is present, as in Fig. 7a, blocking the scatter from reaching the detector, a low resolution image $D_{rl}(i,j)$ is acquired. When the beam selector is absent, as in Fig. 7b, allowing all x-rays to reach the detector, a high resolution image $D_{fh}(x,y)$ is acquired, a portion of which is $D_{fh}(x(i),y(j))$. Here the subscript f denotes that the beam selector 18 is in its transmissive position not in front of the detector 16 and the subscript r denotes that the beam selector 18 is in its blocking position in front of the detector 16. In this special case, when the rest conditions are maintained substantially identical, if, and only if, the x-ray beam transmission through the holes is 100%, an energy-independent constant exists, which is independent of the image subject 12 and is predetermined when the image subject 12 is absent.

$$D_{fPl}(x(i),y(j)) = C_g(i,j) \times D_{rl}(i,j) \qquad (5)$$

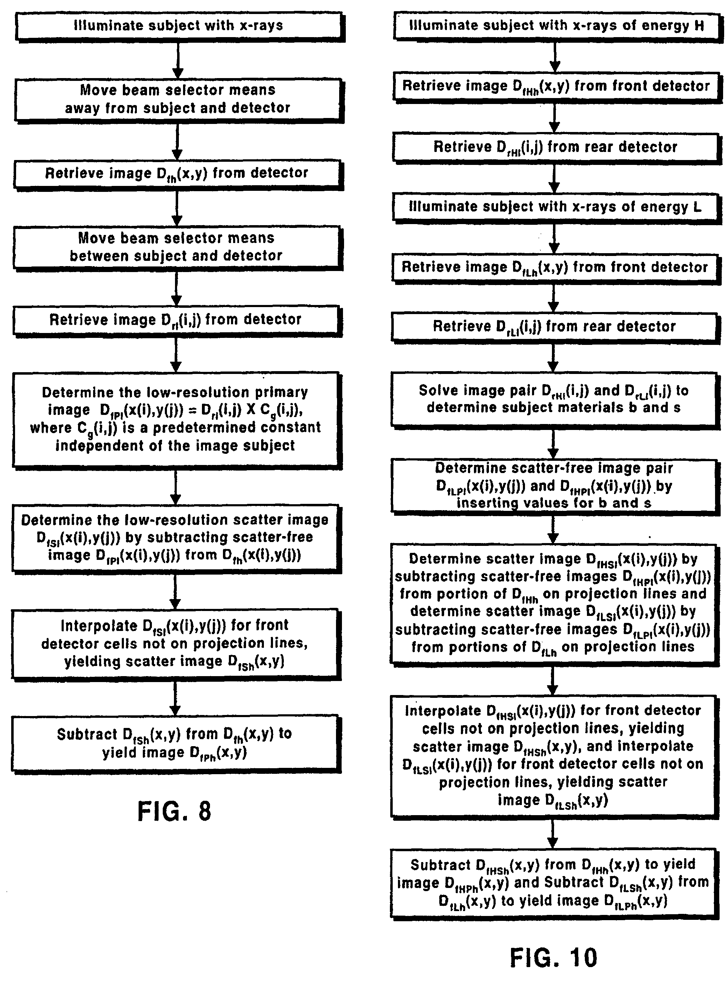

Fig. 8 shows the data processing procedures for the second special case.

<u>Second embodiment</u>

In this embodiment, shown in Fig. 9, the front detector assembly 16 and the rear detector assembly 26 each have only one detector. The x-ray source 14 emits two consecutive pulses, a low-energy pulse followed by a high-energy pulse.

As shown in Fig. 10, the acquired first image pair includes the high-resolution images from the front detector 16 and are

$$D_{fHh}(x,y) = \int [\Phi_{0H}(E) \times \exp(-(\mu_b(E) \times b(x,y) +$$

$$\mu_s(E) \times s(x,y))] \times S_f(E)dE +$$

$$\int \Phi_{fS}(E,x,y) \times S_f(E)dE \qquad (6a)$$

and

$$D_{fLh}(x,y) = \int [\Phi_{0L}(E) \times \exp(-(\mu_b(E) \times b(x,y) +$$

$$\mu_s(E) \times s(x,y))] \times S_f(E)dE +$$

$$\int \Phi_{fS}(E,x,y) \times S_f(E)dE \qquad (6b)$$

and the second image pair includes the low-resolution images from the rear detector 26 and are

$$D_{rHl}(i,j) = \int [\Phi_{0H}(E) \times \exp(-(\mu_b(E) \times b(i,j) +$$

$$\mu_s(E) \times s(i,j))] \times S_r(E)dE \qquad (7a)$$

and

$$D_{rLl}(i,j) = \int [\Phi_{0L}(E) \times \exp(-(\mu_b(E) \times b(i,j) +$$

$$\mu_s(E) \times s(i,j))] \times S_r(E)dE \qquad (7b)$$

In the equation pair 7a, 7b, the acquired low-resolution image data are free of scatter radiation. By using the dual-energy data decomposition methods described below, the simultaneous equation pair 7a, 7b is solved to find the solutions for the image pair of material composition b(i,j) and s(i,j).

As described above with reference to the first embodiment, because the rear detector cell (i,j) and front detector cell (x(i),y(j)) lie on the same selected projection line, the low-resolution front detector primary image pair $D_{fHPl}$(x(i),y(j)), $D_{fLPl}$(x(i),y(j)) can be determined from the rear detector primary image pair $D_{rHl}$(i,j), $D_{rLl}$(i,j). The front detector scatter image pair $D_{fHSl}$(x(i),y(j)), $D_{fLSl}$(x(i),y(j)) are found by the equations

$$D_{fHSl}(x(i),y(j)) = D_{fHl}(x(i),y(j)) - D_{fHPl}(x(i),y(j)) \qquad (8a)$$

and

$$D_{fLSl}(x(i),y(j)) = D_{fLl}(x(i),y(j)) - D_{fLPl}(x(i),y(j)) \qquad (8b)$$

As above, the low-resolution scatter images can be extended to all front detector cells not on the selected projection lines through interpolation without loss of accuracy to yield the high-resolution scatter image pair $D_{fHSh}$(x, y), $D_{fLSh}$(x,y). The high-resolution scatter-free images on the front detector assembly are denoted as $D_{fHPh}$(x,y) and $D_{fLPh}$(x,y) and are

$$D_{fHPh}(x,y) = D_{fHh}(x,y) - D_{fHSh}(x,y) \qquad (9a)$$

and

$$D_{fLPh}(x,y) = D_{fLh}(x,y) - D_{fLSh}(x,y) \qquad (9b)$$

The image pair $D_{fHPh}$(x,y), $D_{fLPh}$(x,y) is a pair of dual-energy x-ray images without scatter. This image pair in turn relates to the material composition of the subject by the equations

$$D_{fHPh}(x,y) = \int [\Phi_{0H}(E) \times \exp(-(\mu_b(E) \times b(x,y) +$$

$$\mu_s(E) \times s(x,y))] \times S_f(E)dE \qquad (10a)$$

and

$$D_{fLPh}(x,y) = \int [\Phi_{0L}(E) \times \exp(-(\mu_b(E) \times b(x,y) +$$

$$\mu_s(E) \times s(x,y))] \times S_f(E)dE \qquad (10b)$$

Thus, in addition to providing one scatter-free image, this embodiment provides a pair of scatter-free dual-energy images in the equation pair 10a, 10b. This equation pair is the fundamental dual-energy x-ray imaging equation system with the unprecedented feature that scatter radiation has been essentially removed from the two-dimensional detector. In the equation pair 10a, 10b, the values of $D_{fLPh}$(x,y) and $D_{fHPh}$(x,y) are known from the above described calculations conducted on the image pair $D_{fHh}$(x,y) $D_{fLh}$(x,y) directly measured from the front detectors 16, and on the image pair $D_{rLl}$(i,j), $D_{rHl}$(i,j) directly measured from the rear detector 26. The unknown values are the two material composition images b(x,y) and s(x,y).

The dual-energy x-ray data decomposition method can be further applied to the equation pair 10a, 10b. As a result, by using the quantitative relationships $b = b(D_H,D_L)$ and $s = s(D_H,D_L)$ provided by the data decomposition method, a pair of high-resolution images b(x,y) and s(x,y) are readily obtained point by point for all front detector cells (x,y). The solution of the two-component material composition images b(x,y) and s(x,y) has a spatial resolution as high as the front detector 16 can provide.

An alternate to the second embodiment substitutes an x-ray source generates x-rays continuously, alternating between high-energy x-rays and low-energy x-rays.

Another alternate to the second embodiment inserts an x-ray energy filter between the x-ray source and the subject at the moment the x-ray source switches to generate the high-energy x-rays. The synchronization between the insertion of the filter and the switching high-voltage or double-pulse is preferably implemented by using a motor drive. The filter absorbs more of the low-energy x-rays, resulting in an increase in the energy difference between the low-energy x-rays and the high-energy x-rays.

This invention not only provides a method and apparatus for removing scattering from two-dimensional detectors, but at the same time also provides a method and apparatus for scatter-free dual-energy x-ray imaging using two-dimensional detectors.

The interrelationship between the method for removing scatter radiation in two-dimensional detectors and the method for dual-energy x-ray imaging using two-dimensional detectors can be summarized as follows:

1. The method for removing scatter radiation from two-dimensional detectors utilizes and hinges on the method of dual-energy x-ray imaging free of scatter. Without dual-energy x-ray imaging, the scatter radiation cannot be accurately removed.

2. The method for dual-energy x-ray imaging using two-dimensional detectors utilizes and hinges on the method of removing scatter radiation. Without substantially removing scatter from two-dimensional detectors, the accuracy of dual-energy x-ray imaging would be so degraded as to be meaningless. This invention solves both problems in a unified system.

Third Embodiment

The third embodiment, uses a pair of two-dimensional detector assemblies. The x-ray source 14 is a constant potential x-ray source that emits steady state x-rays, single pulse x-rays, or repetitive pulse x-rays with the same energy spectrum. The front detector assembly 16 has a low-energy two-dimensional detector 50, an x-ray energy spectral filter 52, and a high-energy two-dimensional detector 54. The rear detector assembly 26 also has a low-energy two-dimensional detector 56, an x-ray energy spectral filter 58, and a high-energy two-dimensional detector 60. The filters 52, 58 operate in the conventional manner as described above with reference to the first embodiment. The front high-energy detector 54 is sensitive to higher x-ray energies than the front low-energy detector 50 and the rear high-energy detector 60 is sensitive to higher x-ray energies than the rear low-energy detector 56.

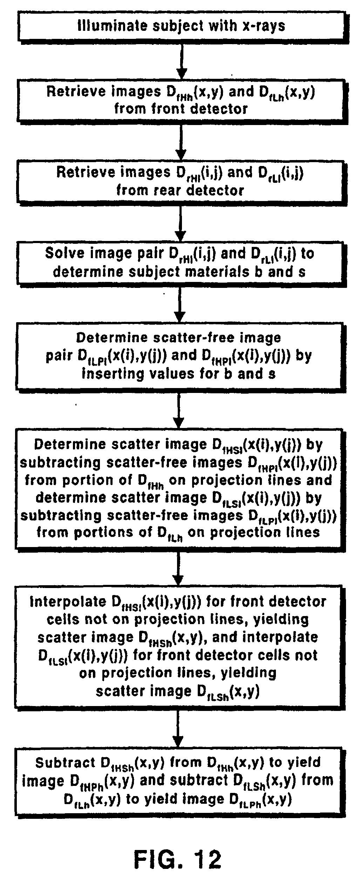

This embodiment requires only single-pulse, constant-energy x-rays, as in the first embodiment, rather than the dual-energy x-ray source of the second embodiment. As shown in Fig. 12, following a single-pulse x-ray, two pairs of dual-energy x-ray images are acquired. The first pair includes the high-resolution images $D_{fLh}(x,y)$ from the low-energy front detector 50 and $D_{fHh}(x,y)$ from the high-energy front detector 54.

The second pair includes the low-resolution images $D_{rLl}(i,j)$ from the low-energy rear detector 56 and $D_{rHl}(i,j)$ from the high-energy rear detector 60.

As described above with relation to the second embodiment, the image pair $D_{rLl}(i,j)$ and $D_{rHl}(i,j)$ does not contain scatter. Thus, a pair of material composition images b(i,j), s(i,j) can be calculated for each cell in the (i,j) plane. This image pair is used to first determine the low-resolution primary image pair $D_{fHPl}(x(i), y(j))$, $D_{fHPl}(x(i),y(j))$ and then to determine scatter image pair $D_{fHSl}(x(i),y(j))$, $D_{fLSl}(x(i),y(j))$ in the same way as for the equation pair 14a, 14b. Scatter image pair $D_{fHSl}(x(i),y(j))$, $D_{fLSl}(x(i),y(j))$ is used to further determine the high-resolution scatter image pair $D_{fHSh}(x,y)$, $D_{fLSh}(x,y)$, as described above in the second embodiment. By subtracting the calculated high-resolution scatter image pair $D_{fHSh}(x,y)$, $D_{fLSh}(x,y)$ from the front detector 16 high-resolution image pair $D_{fHh}(x,y)$, $D_{fLh}(x,y)$, the high spatial resolution image pair free of scatter $D_{fHPh}(x,y)$ and $D_{fLPh}(x,y)$ is numerically obtained. Using the inversion method described below, a pair of high-accuracy, high-resolution material composition images b(x,y) and s(x,y) can be further obtained.

Data Decomposition Method

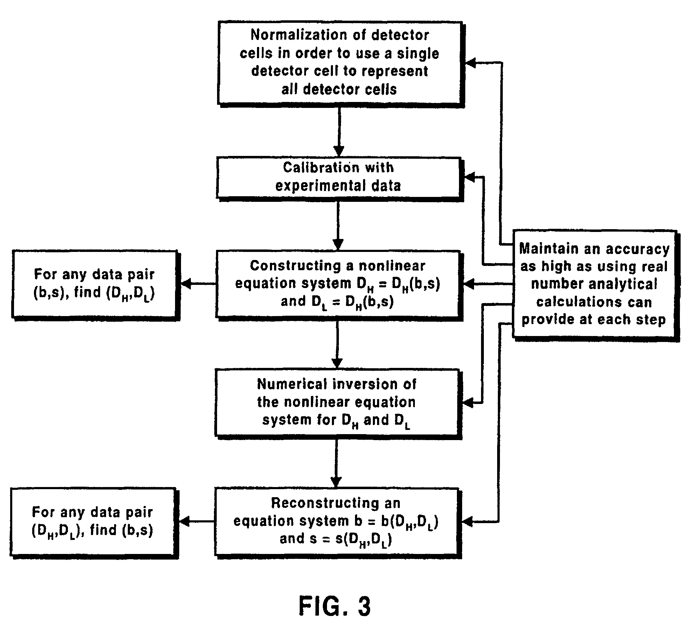

The following is a step-by-step description of the data decomposition method summarized above and shown in Fig. 3.

The first step is to construct the two simultaneous numerical equations $D_L = D_L(b,s)$ and $D_H = D_H(b,s)$ in three-dimensional space. A preferred method for doing this is to determine the function S(E) through the standard absorption method.

An absorption curve is measured by using a collimated narrow primary x-ray beam. The electrical signal from a single detector cell D(t) as a function of the absorption plate thickness t is experimentally determined and is related to S(E) through the equation

$$D(t)= \int S(E) \times \Phi_0(E) \times \exp(-\mu(E) \times t)dE \tag{11}$$

Since the mass absorption coefficient $\mu(E)$ of the absorption plate material is known, the function S(E) can be determined. This method is especially convenient for the internal conversion type of two-dimensional x-ray detectors. In these detectors, the detection efficiency and detector energy response function can be expressed in a simple analytical expression with few unknown parameters to be solved,

$$S(E) = \{S_0[1-\exp(-\mu_0(E) \times d)] \times \alpha E\} \times$$

$$\exp(-\mu_1(E) \times d_1 - \mu_2(E) \times d_2) \tag{12}$$

where $S_0(E)= [1-\exp(-\mu_0(E) \times d)] \times \alpha E$ is the electrical signal amplitude induced by x-ray photons with energy E, $\mu_0$(E) is the mass absorption coefficient of the detector's conversion layer, d is the thickness of the conversion layer of the detector cell, and where $S_1(E) = \exp(-\mu_1(E) \times d_1 - \mu_2(E) \times d_2)$ is the x-ray transmission after leaving the image subject to the detector surface, $\mu_1(E)$ and $\mu_2(E)$ are the absorption coefficients of two given materials, and $d_1$ and $d_2$ are the thickness values of these materials.

When the x-ray energy spectrum $\Phi_0(E)$ is separately measured, these unknown parameters $\alpha$, d, $d_1$, and $d_2$ are determined by using standard least square parameter-fitting techniques through equation 12. Then, the function S(E) is obtained to a high degree of accuracy for a single cell.

Once the value for S(E) is determined to the desired accuracy, the dual-energy signals as a function of the material composition of the subject can be calculated through the equations

$$D_H = \int S(E) \times \Phi_0(E)x \exp(-(\mu_b(E) \times b +$$

$$\mu s(E) \times s)dE \tag{13a}$$

and

$$D_L = \int S(E) \times \Phi_0(E)x \exp(-(\mu_b(E) \times b +$$

$$\mu_s(E) \times s)dE \tag{13b}$$

where $\mu_b(E)$ and $\mu_s(E)$ are the well-documented mass absorption coefficients for bone tissue and soft tissue, respectively. The mass surface densities b and s are assigned values that sufficiently cover the real range of the subject 12.

Another preferred method for constructing the quantitative explicit functions $D_L = D_L(b,s)$ and $D_H = D_H(b,s)$ is to conduct direct measurements of signals $D_L$ and $D_H$ at a number of selected b and s values. The entire functions $D_L = D_L(b,s)$ and $D_H = D_H(b,s)$ are obtained from the directly measured data points by using standard two-dimensional interpolation algorithms. The interpolation in this case is valid because the functions $D_L = D_L(b,s)$ and $D_H = D_H(b,s)$ are continuous, smooth, and monotonous.

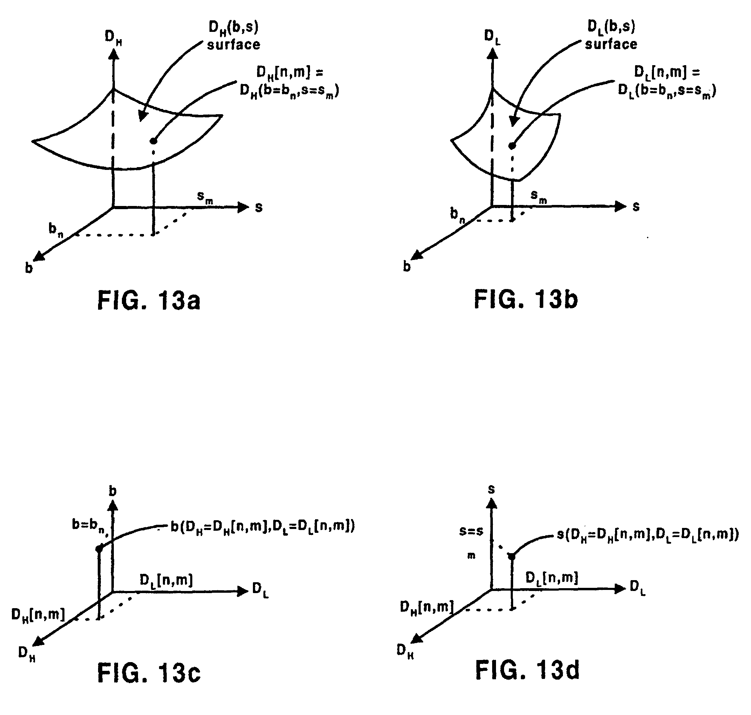

The second step is to determine the material composition images b and s as functions of the image pair $D_H$, $D_L$. The procedures for obtaining a simultaneous equation system for $b(D_H,D_L)$ and $s(D_H,D_L)$ are shown graphically in Figs. 13a to 13d. To do so, the simultaneous equation pair 24a, 24b must be inverted. A preferred method of inversion is as follows: (1) as in Figs. 13a and 13b, assign a pair of values in the desired range to b and s corresponding to one of the coordinate points in the (b,s) plane so that $b = b_n$, and $s = s_m$, where n = 0,1,2...N, and m = 0,1,2...M. Typical N and M values are in the range of between approximately 50 and approximately 5,000. The larger N and M, the higher the accuracy of the results. However, the largest values for N and M are limited by the available capacity of computer memory and computing speed. From the two numerical equations representing the three-dimensional surfaces $D_H(b, s)$ and $D_L(b,s)$, determine a pair of $D_H$ and $D_L$ values so that $D_H[n,m] = D_H(b = b_n, s = s_m)$ and $D_L[n,m] = D_L(b = b_n, s = s_m)$, where $D_H[n,m]$ and $D_L[n,m]$ are two specific real numbers, and (2) as in Figs. 13c and 13d, replot the four numbers $D_H[n,m]$, $D_L[n,m]$, $b_n$, and $s_m$ to provide a pair of data points on the three-dimensional surfaces $b(D_H,D_L)$ and $s(D_H,D_L)$. The data point on the three-dimensional surface $b(D_H,D_L)$ is $D_H = D_H[n,m]$, $D_L = D_L[n,m]$, $b = b_n$, and the data point on the three-dimensional surface $s(D_H,D_L)$ is $D_H = D_H[n,m]$, $D_L = D_L[n,m]$, $s = s_m$. After going through all the $b = b_n$ values $(b_0,b_1,b_2,...,b_N)$ and all the $s = s_m$ values $(s_0,s_1,s_2,...,s_M)$, the essential part of the inversion task is complete. However, for the purpose of storing the inverted arrays $b = b(D_H,D_L)$ and $s = s(D_H,D_L)$, the step sizes of $D_H = D_H[n,m]$ and $D_L = D_L[n,m]$ must be adjusted. In the inverted space, $D_H$ and $D_L$ are basis coordinates. From the N x M data points, only J data points are selected for $D_H$ and only K data points are selected for $D_L$, where J and K are approximately in the same range as N and M. In the final form after the second step, two two-dimensional arrays are obtained and stored: $b = b(D_H,D_L)$ and $s = s(D_H,D_L)$, where $D_H = D_H[j]$, $D_L = D_L[k]$; j=0,1,2,...,J, $D_H[j] > D_H[j+1]$ and k=0,1,2,...,K, $D_L[k] > D_L[k+1]$. Two additional one-dimensional arrays $D_H[j]$ and $D_L[k]$ are also stored. Arrays $D_H[j]$ and $D_L[k]$ are saved so that accuracy as high as real number calculations can provide is maintained.

The third step is to find the desired results from the input data according to the established equations. The desired values for b and s at each cell location is determined by inserting the available data pair $(D_H,D_L)$ into the numerical equations of step 2. Conversely, the desired values for $D_H$, $D_L$, or only one of them if only one is needed, at each discrete cell location is determined by inserting the available data pair (b,s) into the numerical equations of step 1.

The final step is to maintain the accuracy of the values for b and s in order to maintain a continuous domain function. The following procedures ensure elimination of the errors in connection with finite steps in data processing.

In step 1, in the process of constructing the equation pair for $D_H[n,m] = D_H(b = b_n, s = s_m)$ and $D_L[n,m] = D_L(b = b_n, s = s_m)$, for each pair of values of $b_n$ and $s_m$, the $D_H[n,m]$ and $D_L[n,m]$ are measured or calculated to an accuracy of real numbers. $D_H[n,m]$ and $D_L[n,m]$ are stored in computer as real number arrays.

In step 2, the inversion process, including replotting in $D_H$ space and $D_L$ space, introduces no errors due to the data processing. The step sizes can be changed without losing any accuracy as long as values for $D_H = D_H[j]$ are selected that are exactly equal to one of the $D_H[n,m]$ values that satisfies the condition $D_H[j-1] > D_H[j] > D_H[j+1]$, and values for $D_L = D_L[k]$ are selected that are exactly equal to one of the $D_L[n,m]$ values that satisfies the condition $D_L[k-1] > D_L[k] > D_L[k+1]$.

In step 3, for each measured dual-energy signal data pair $(D_{HEX}, D_{LEX})$, first to find out the closest j and k values according to the criteria: $D_H[j] \geq D_{HEX} \geq D_H[j+1]$ and $D_L[k] \geq D_{LEX} \geq D_L[k+1]$. From the index values j and k, the closest b and s are first determined as $b_0 = b_0(D_H[j], D_L[k])$ and $s_0 = s_0(D_H[j], D_L[k])$. The following equations give b and s values to an accuracy as high as real number calculations can provide:

$$b = b_0(D_H[j],D_L[k]) + [\partial b(D_H, D_L) / \partial D_L]_{DH=DH[j]; \, DL=DL[k]} \times$$

$$(D_{LEX} - D_L[k]) + [\partial b(D_H,D_L)/\partial D_H]_{DH=DH[j]; \, DL=DL[k]} \times$$

$$(D_{HEX} - D_H[j]) + \text{higher order terms} \qquad (14a)$$

and

$$s = s_0(D_H[j], D_L[k]) + [\partial s(D_H,D_L) / \partial D_L]_{DH=DH(j); \, DL=DL[k]} \times$$

$$(D_{LEX} - D_L[k]) + [\partial s(D_H, D_L)/\partial D_H]_{DH=DH[j]; \, DL=DL[k]} \times$$

$$(D_{HEX} - D_H[j]) + \text{higher order terms} \qquad (14b)$$

where the values for the higher order terms are found in standard calculus textbooks.

Also in step 3, if the image pair $D_H$ and $D_L$ from a given material composition data pair $(b_{ex},s_{ex})$ is to be found, $D_H$ and $D_L$ is obtained to an accuracy of real numbers by using similar standard Taylor expressions.

Thus, the procedures described above provide methods for directly solving the nonlinear dual-energy x-ray imaging fundamental equation systems in its original form with arbitrary bremsstrahlung spectra at an accuracy as high as using real number analytical calculations can provide.

The following is a list of possible further variations in the embodiments:

(1) Some well-established computation tools, such as sorting algorithms or database procedures, can be used to carry out the inversion process described above. The use of any such software package to conduct the above-described inversion process is contemplated by the present invention.

(2) All of the steps described above, including the data decomposition method and the scatter elimination method, can be combined together to various degrees, from combining any two steps to combining all the steps into one procedure. For example, image pair $(D_{rH}, D_{rL})$ can be used directly to find $(D_{fHP}, D_{fLP})$ without explicitly determining (b,s). One way of doing so is to construct a pair of quantitative relationships $D_{fHP} = (D_{rH}, D_{rL})$ and $D_{fLP} = (D_{rH}, D_{rL})$ in a data base and storing them. Any such combination methods are contemplated by the present invention.

It is intended that the scope of the invention be limited not by this detailed description, but rather by the claims appended hereto.

**Claims**

1. A two-dimensional x-ray imaging system for taking images of a subject (12), said system comprising:

   (a) an x-ray source (14) adapted to emit x-rays (30) for passage through said subject (12), said x-rays (30) including primary x-rays (32) having their direction of travel unaltered by interaction with said subject (12) and said x-rays (30) including scatter x-rays (34) having their direction of travel altered by interaction with said subject (12);
   (b) x-ray detection means for detecting said x-rays in two dimensions,
   (c) beam selection means (18) for blocking passage of substantially all of said scatter x-rays (34) and permitting passage of a portion of said primary x-rays (32) ;

   wherein said subject (12) is located between said x-ray source (14) and said x-ray detection means; and
   said detection means includes high-resolution detection means for detecting said primary x-rays (32) and said scatter x-rays (34) simultaneously, and low-resolution detection means for detecting only said x-rays (30) passed by said beam selection means (18).

2. An x-ray imaging system according to claim 1, wherein said beam selection means (18) is substantially composed of an x-ray-absorbent material having a plurality of holes (20), the axes of said holes (20) being parallel to the direction of travel of said primary x-rays (32).

3. An x-ray imaging system according to claim 1 or 2, wherein said detection means includes a detector assembly (16) and wherein said beam selection means (18) has two positions, a transmissive position wherein said detector assembly acts as said high-resolution detection means, and a blocking position wherein said detector assembly acts as said low-resolution detection means.

4. An x-ray imaging system according to claim 1 or 2, wherein said high-resolution detection means includes a front detector assembly (16) and said low-resolution detection means includes a rear detector assembly (26), and wherein said system comprises, in physical sequence from front to rear, said x-ray source (14), said front detector assembly (16), said beam selection means (18), and said rear detector assembly (26), said subject (12) being located between said x-ray source (14) and said front detector assembly (16).

5. An x-ray imaging system according to any preceding claim, wherein said x-ray source (14) is adapted to emit x-ray pulses of two different average energies.

6. An x-ray imaging system according to claim 4 and 5, wherein said rear detector assembly (26) includes, in physical sequence from front to rear, a rear low-energy detector (40), a rear x-ray energy spectral filter (42), and a rear high-energy detector (44), said rear low-energy detector (40) including a plurality of x-ray-sensitive detector cells arranged in a substantially rectangular matrix, said rear high-energy detector (44) including a plurality of x-ray-sensitive detector cells, the arrangement and quantity of said rear high-energy detector cells being substantially the same as the arrangement and quantity of said rear low-energy detector cells.

7. An x-ray imaging system according to claim 6, wherein said front detector assembly (16) includes, in physical sequence from front to rear, a front low-energy detector (50), a front x-ray energy spectral filter (52), and a front high-energy detector (54), said front low-energy detector (50) including a plurality of x-ray-sensitive detector cells arranged in a substantially rectangular matrix, said front high-energy detector (54) including a plurality of x-ray-sensitive detector cells, the arrangement and quantity of said front high-energy detector cells being substantially the same as the arrangement and quantity of said front low-energy detector cells.

8. A method for taking a two-dimensional x-ray image of a subject (12) using a two-dimensional x-ray imaging system, said subject (12) being composed substantially of two materials that interact differently with x-rays (30), said imaging system including an x-ray source (14), beam selection means (18), and a two-dimensional x-ray detector assembly having a plurality of detection locations, said subject (12) being between said x-ray source (14) and said detector assembly, said x-ray source (14) being adapted to emit x-rays (30) for passage through said subject (12), including primary x-rays (32) having their direction of travel unaltered by interaction with said subject (12), and scatter x-rays (34) having their direction of travel altered by interaction with said subject (12), said beam selection means (18) selectively allowing substantially only primary x-rays (32) to reach selected detection locations, said method comprising:

(a) illuminating said subject (12) with said x-rays (30) ;

(b) acquiring a raw high-resolution image $I_{fh}$ from said detection locations and processing said image $I_{fh}$ to normalize it and to subtract dark signals, yielding a high-resolution image $D_{fh}$ composed of said primary x-rays (32) and said scatter x-rays (34);

(c) producing, from said image $D_{fh}$, a low-resolution image $D_{fl}$ corresponding to said selected detection locations;

(d) acquiring a raw low-resolution image $I_{rl}$ from said selected detection locations and processing said image $I_{rl}$ to normalize it and to subtract dark signals, yielding a low-resolution image $D_{rl}$ composed of substantially only said primary x-rays (32);

(e) calculating a low-resolution primary x-ray image $D_{fPl}$ corresponding to said selected detection locations by multiplying said image $D_{rl}$ by a predetermined factor that is independent of said subject (12);

(f) calculating a low-resolution scatter x-ray image $D_{fSl}$ corresponding to said selected detection locations by subtracting said image $D_{fPl}$ from said image $D_{fl}$;

(g) calculating a high-resolution scatter x-ray image $D_{fSh}$ by interpolation from said low-resolution scatter x-ray image $D_{fSl}$; and

(h) calculating a high-resolution primary x-ray image $D_{fPh}$ by subtracting said high-resolution scatter x-ray image $D_{fSh}$ from said high-resolution image $D_{fh}$;

whereby said image $D_{fPh}$ is a high-resolution, two-dimensional primary x-ray (32) image of said subject (12) after said scatter x-rays (34) have been substantially eliminated, said image $D_{fPh}$ having a resolution substantially equal to the highest resolution available from said detector assembly.

9. A method according to claim 8, wherein:

(a) said beam selection means (18) is moveable between a blocking position that allows substantially only primary x-rays.(32) to reach said selected detection locations and a transmissive position that allows both said primary x-rays (32) and said scatter x-rays (34) to reach said selected detection locations;

(b) said beam selection means (18) is moved to said transmissive position prior to acquiring said raw high-resolution image $I_{fh}$; and

(c) said beam selection means (18) is moved to said blocking position prior to acquiring said raw low-resolution image $I_{rl}$.

10. A method according to claim 8, wherein said x-ray detector assembly includes a front two-dimensional x-ray detector assembly (16) and a rear two-dimensional x-ray detector assembly (26), said beam selection means (18) positioned therebetween, said high-resolution image $I_{fh}$ is acquired from front detection locations on said front detector assembly (16), and said selected detection locations are selected rear detection locations on said rear detector assembly (26) receiving substantially only said primary x-rays (32) passed by said beam selection means (18), said low-resolution image $D_{fl}$ is produced from front detection locations intersected by x-ray projection lines extending from said x-ray source (14) to said selected rear detection locations.

11. A method according to claim 10, wherein:

(a) said x-ray source (14) is adapted to emit x-rays (30) at average energy level L at time $t_L$ and at average energy level H at time $t_H$;

(b) said raw high-resolution image $I_{fh}$ consists of a low-energy image $I_{fLh}$ acquired from said front detection locations at time $t_L$ and a high-energy image $I_{fHh}$ acquired from said front detection locations at time $t_H$, and said image $D_{fh}$ consists of a low-energy image $D_{fLh}$ calculated by processing said raw low-energy image $I_{fLh}$ to normalize it and to subtract dark signals, and a high-energy image $D_{fHh}$ calculated by processing said raw high-energy image $I_{fHh}$ to normalize it and to subtract dark signals;

(c) said low-resolution image $D_{fl}$ consists of a low-resolution image $D_{fLl}$ corresponding to said selected rear detection locations and produced from said image $D_{fLh}$, and a low-resolution image $D_{fHl}$ corresponding to said selected rear detection locations and produced from said image $D_{fHh}$;

(d) said raw low-resolution image $I_{rl}$ consists of a low-energy image $I_{rLl}$ acquired from said selected rear detection locations at time $t_L$ and a high-energy image $I_{rHl}$ acquired from said selected rear detection locations at time $t_H$, and said image $D_{rl}$ consists of a low-energy image $D_{rLl}$ calculated by processing said image $I_{rLl}$ to normalize it and to subtract dark signals, and a high-energy image $D_{rHl}$ calculated by processing said image $I_{rHl}$ to normalize it and to subtract dark signals;

(e) said primary x-ray image $D_{fPl}$ consists of an image $D_{fLPl}$ calculated by multiplying said image $D_{rLl}$ by a predetermined low-energy factor, and an image $D_{fHPl}$ calculated by multiplying said image $D_{rHl}$ by a predetermined high-energy factor;

(f) said low-resolution scatter x-ray image $D_{fSl}$ consists of an image $D_{fLSl}$ calculated by subtracting said image $D_{fLPl}$ from said image $D_{fLl}$, and an image $D_{fHSl}$ calculated by subtracting said image $D_{fHPl}$ from said image $D_{fHl}$;

(g) said high-resolution scatter x-ray image $D_{fSh}$ consists of a high-resolution scatter x-ray image $D_{fLSh}$ calculated by interpolation from said low-resolution scatter x-ray image $D_{fLSl}$, and a high-resolution scatter x-ray image $D_{fHSh}$ calculated by interpolation from said low-resolution scatter x-ray image $D_{fHSl}$; and

(h) said high-resolution primary x-ray image $D_{fPh}$ consists of a high-resolution primary x-ray image $D_{fLPh}$ calculated by subtracting said image $D_{fLSh}$ from said image $D_{fLh}$, and a high-resolution primary x-ray image $D_{fHPh}$ calculated by subtracting said image $D_{fHSh}$ from said image $D_{fHh}$;

whereby said images $D_{fLPh}$ and $D_{fHPh}$ form a high-resolution, two-dimensional, dual-energy primary x-ray image pair $D_{fLPh}$, $D_{fHPh}$ of said subject (12) after said scatter x-rays (34) have been substantially eliminated, said image pair having a resolution substantially equal to the highest resolution available from said front detector assembly (16).

**12.** A method according to claim 10, wherein:

(a) said x-ray source (14) is adapted to emit x-rays (30) at an average energy level L and at an average energy level H simultaneously, said rear detector assembly (26) includes, in physical order, a rear low-energy x-ray detector (40), a rear x-ray energy spectral filter (42), and a rear high-energy x-ray detector (44), said selected rear detection locations including selected rear low-energy detection locations at said rear low-energy detector (40) and corresponding rear high-energy detection locations at said rear high-energy detector (44);

(b) said raw low-resolution image $I_{rl}$ consists of a low-energy image $I_{rLl}$ acquired from said selected rear low-energy detection locations and a high-energy image $I_{rHl}$ acquired from said selected rear high-energy detection locations, and said image $D_{rl}$ consists of a low-energy image $D_{rLl}$ calculated by processing said image $I_{rLl}$ to normalize it and to subtract dark signals, and a high-energy image $D_{rHl}$ calculated by processing said image $I_{rHl}$ to normalize it and to subtract dark signals; and

(c) said low-resolution primary x-ray image $D_{fPl}$ is calculated from said images $D_{rLl}$ and $D_{rHl}$.

**13.** A method according to claim 12, wherein:

(a) said front detector assembly (16) includes, in physical order, a front low-energy x-ray detector (50), a front x-ray energy spectral filter (52), and a front high-energy x-ray detector (54);

(b) said raw high-resolution image $I_{fh}$ consists of a low-energy image $I_{fLh}$ acquired from said front low-energy detection locations and a high-energy image $I_{fHh}$ acquired from said front high-energy detection locations, and said high-resolution image $D_{fh}$ consists of a low-energy image $D_{fLh}$ calculated by processing said image $I_{fLh}$ to normalize it and to subtract dark signals, and a high-energy image $D_{fHh}$ calculated by processing said image $I_{fHh}$ to normalize it and to subtract dark signals;

(c) said low-resolution image $D_{fl}$ consists of a low-resolution image $D_{fLl}$ corresponding to said selected rear low-energy detection locations and produced from said low-energy image $D_{fLh}$, and a low-resolution image $D_{fHl}$ corresponding to selected rear high-energy detection locations and produced from said high-energy image $D_{fHh}$;

(d) said primary x-ray image $D_{fPl}$ consists of an image $D_{fLPl}$ calculated by multiplying said low-energy image

$D_{rLI}$ by a predetermined low-energy factor, and an image $D_{fHPI}$ calculated by multiplying said high-energy image $D_{rHI}$ by a predetermined high-energy factor;

(e) said low-resolution scatter x-ray image $D_{fSI}$ consists of an image $D_{fLSI}$ calculated by subtracting said multiplied low-energy image $D_{fLPI}$ from said low-energy low-resolution image $D_{fLI}$, and an image $D_{fHSI}$ calculated by subtracting said multiplied high-energy image $D_{fHPI}$ from said high-energy low-resolution image $D_{fHI}$;

(f) said high-resolution scatter x-ray image $D_{fSh}$ consists of a high-resolution scatter x-ray image $D_{fLSh}$ calculated by interpolation from said low-resolution scatter x-ray image $D_{fLSI}$, and a high-resolution scatter x-ray image $D_{fHSh}$ calculated by interpolation from said low-resolution scatter x-ray image $D_{fHSI}$; and

(g) said high-resolution primary x-ray image $D_{fPh}$ consists of a high-resolution primary x-ray image $D_{fLPh}$ calculated by subtracting said low-energy scatter image $D_{fLSh}$ from said low-energy image $D_{fLh}$, and a high-resolution primary x-ray image $D_{fHPh}$ calculated by subtracting said high-energy scatter image $D_{fHSh}$ from said high-energy image $D_{fHh}$;

whereby said images $D_{fLPh}$ and $D_{fHph}$ form a high-resolution, two-dimensional, dual-energy primary x-ray image pair $D_{fLPh}$, $D_{fHPh}$ of said subject (12) after said scatter x-rays (34) have been substantially eliminated, said image pair having a resolution substantially equal to the highest resolution available from said front detector assembly (16).

## Patentansprüche

1. Zweidimensionales Röntgen-Bildgebersystem, das Aufnahmen von einem Subjekt (12) erzeugt, umfassend:

   a) eine Röntgenstrahlenquelle (14), die dafür ausgelegt ist, Röntgenstrahlen (30) für den Durchgang durch das Subjekt (12) auszusenden, wobei die Röntgenstrahlen (30) Hauptröntgenstrahlen (32) enthalten, deren Ausbreitungsrichtung durch die Wechselwirkung mit dem Subjekt (12) nicht verändert wird, und die Röntgenstrahlen (30) Streuröntgenstrahlen (34) enthalten, deren Ausbreitungsrichtung durch die Wechselwirkung mit dem Subjekt (12) verändert wird;
   b) eineRöntgenstrahlen-Erfassungsvorrichtung, die die Röntgenstrahlen in zwei Dimensionen erfasst;
   c) eine Strahlauswahlvorrichtung (18), die den Durchgang im Wesentlichen aller Streuröntgenstrahlen (34) verhindert und den Durchgang eines Teils der Hauptröntgenstrahlen (32) erlaubt,

   wobei sich das Subjekt (12) zwischen der Röntgenstrahlenquelle (14) und der Röntgenstrahlen-Erfassungsvorrichtung befindet, und
   die Erfassungsvorrichtung eine hoch auflösende Erfassungsvorrichtung enthält, die die Hauptröntgenstrahlen (32) und die Streuröntgenstrahlen (34) simultan erfasst, und eine niedrig auflösende Erfassungsvorrichtung, die nur Röntgenstrahlen (30) erfasst, die von der Strahlauswahlvorrichtung (18) durchgelassen werden.

2. Röntgen-Bildgebersystem nach Anspruch 1, wobei die Strahlauswahlvorrichtung (18) im Wesentlichen aus einem Röntgenstrahlen absorbierenden Material aufgebaut ist, das zahlreiche Löcher (20) aufweist, und die Achsen der Löcher (20) parallel zur Ausbreitungsrichtung der Hauptröntgenstrahlen (32) sind.

3. Röntgen-Bildgebersystem nach Anspruch 1 oder 2, wobei die Erfassungsvorrichtung eine Detektorbaugruppe (16) enthält und die Strahlauswahlvorrichtung (18) zwei Positionen besitzt, nämlich eine durchlässige Position, in der die Detektorbaugruppe als hoch auflösende Detektorvorrichtung wirkt, und eine Blockierposition, in der die Detektorbaugruppe als niedrig auflösende Detektor Vorrichtung wirkt.

4. Röntgen-Bildgebersystem nach Anspruch 1 oder 2, wobei die hoch auflösende Erfassungsvorrichtung eine vordere Detektorbaugruppe (16) enthält und die niedrig auflösende Erfassungsvorrichtung eine rückwärtige Detektorbaugruppe (26) enthält, und das System in körperlicher Abfolge von vorne nach hinten die Röntgenstrahlenquelle (14), die vordere Detektorbaugruppe (16), die Strahlauswahlvorrichtung (18) und die rückwärtige Detektorbaugruppe (26) umfasst, und sich das Subjekt (12) zwischen der Röntgenstrahlenquelle (14) und der vorderen Detektorbaugruppe (16) befindet.

5. Röntgen-Bildgebersystem nach irgendeinem vorhergehenden Anspruch, worin die Röntgenstrahlenquelle (14) so eingerichtet ist, dass sie Röntgenstrahlenimpulse mit zwei unterschiedlichen mittleren Energien aussendet.

6. Röntgen-Bildgebersystem nach Anspruch 4 und 5, worin die rückwärtige Detektorbaugruppe (26) in körperlicher Abfolge von vorne nach hinten einen rückwärtigen Niederenergiedetektor (40), ein rückwärtiges Röntgenstrahlenenergie-Spektralfilter (42) und einen rückwärtigen Hochenergiedetektor (44) umfasst, und der rückwärtige Niederenergiedetektor (40) zahlreiche auf Röntgenstrahlen ansprechende Detektorzellen enthält, die in einer im Wesentlichen rechteckigen Matrix angeordnet sind, und der rückwärtige Hochenergiedetektor (44) zahlreiche auf Röntgenstrahlen ansprechende Detektorzellen enthält, und die Anordnung und Anzahl der rückwärtigen Hochenergie-Detektorzellen im Wesentlichen der Anordnung und Anzahl der rückwärtigen Niederenergie-Detektorzellen gleicht.

**7.** Röntgen-Bildgebersystem nach Anspruch 6, worin die vordere Detektorbaugruppe (16) in körperlicher Abfolge von vorne nach hinten einen vorderen Niederenergiedetektor (50), ein vorderes Röntgenstrahlenenergie-Spektralfilter (52) und einen vorderen Hochenergiedetektor (54) umfasst, und der vordere Niederenergiedetektor (50) zahlreiche auf Röntgenstrahlen ansprechende Detektorzellen enthält, die in einer im Wesentlichen rechteckigen Matrix angeordnet sind, und der vordere Hochenergiedetektor (54) zahlreiche auf Röntgenstrahlen ansprechende Detektorzellen enthält, und die Anordnung und Anzahl der vorderen Hochenergie-Detektorzellen im Wesentlichen der Anordnung und Anzahl der vorderen Niederenergie-Detektorzellen gleicht.

**8.** Verfahren zum Aufnehmen eines zweidimensionalen Röntgenbilds eines Subjekts (12) mit Hilfe eines zweidimensionalen Röntgen-Bildgebersystems, wobei das Subjekt (12) im Wesentlichen aus zwei Materialien zusammengesetzt ist, die unterschiedlich mit Röntgenstrahlen (30) wechselwirken, und das Bildgebersystem eine Röntgenstrahlenquelle (14), eine Strahlauswahlvorrichtung (18) und eine zweidimensionale Röntgenstrahlen-Detektorbaugruppe enthält, die zahlreiche Erfassungsstellen aufweist, und sich das Subjekt (12) zwischen der Röntgenstrahlenquelle (14) und der Detektorbaugruppe befindet, und die Röntgenstrahlenquelle (14) dafür eingerichtet ist, Röntgenstrahlen (30) für den Durchgang durch das Subjekt (12) auszusenden, die Hauptröntgenstrahlen (32) enthalten, deren Ausbreitungsrichtung durch die Wechselwirkung mit dem Subjekt (12) nicht verändert wird, und Streuröntgenstrahlen (34), deren Ausbreitungsrichtung durch die Wechselwirkung mit dem Subjekt (12) verändert wird, und es die Strahlauswahlvorrichtung (18) gezielt im Wesentlichen nur den Hauptröntgenstrahlen (32) ermöglicht, die ausgewählten Erfassungsstellen zu erreichen, und das Verfahren umfasst:

a) Bestrahlen des Subjekts (12) mit den Röntgenstrahlen (30);
b) Erfassen eines groben hoch auflösenden Bilds $I_{fh}$ von den Erfassungsstellen und Verarbeiten des Bilds $I_{fh}$, um es zu normieren und dunkle Signale zu subtrahieren, damit man ein hoch auflösendes Bild $D_{fh}$ erhält, das aus den Hauptröntgenstrahlen (32) und den Streuröntgenstrahlen (34) zusammengesetzt ist;
c) Erzeugen, und zwar aus dem Bild $D_{fh}$, eines niedrig auflösenden Bilds $D_{fl}$, das zu den gewählten Erfassungsstellen gehört;
d) Gewinnen eines niedrig auflösenden Rohbilds $I_{rl}$ von den gewählten Erfassungsstellen und Verarbeiten des Bilds $I_{rl}$, damit es normiert wird und die dunklen Signale entfernt werden, so dass man ein niedrig auflösendes Bild $D_{rl}$ erhält, das im Wesentlichen nur aus den Hauptröntgenstrahlen (32) aufgebaut ist;
e) Berechnen eines niedrig auflösenden Hauptröntgenbilds $D_{fPl}$ entsprechend den gewählten Erfassungsstellen durch das Multiplizieren des Bilds $D_{rl}$ mit einem vorbestimmten Faktor, der vom Subjekt (12) unabhängig ist;
f) Berechnen eines niedrig auflösenden Streuröntgenbilds $D_{fSl}$ entsprechend den gewählten Erfassungsstellen durch das Subtrahieren des Bilds $D_{fPl}$ vom Bild $D_{fl}$;
g) Berechnen eines hoch auflösenden Streuröntgenbilds $D_{fSh}$ durch Interpolation aus dem niedrig auflösenden Streuröntgenbild $D_{fSl}$; und
h) Berechnen eines hoch auflösenden Hauptröntgenbilds $D_{fPh}$ durch das Subtrahieren des hoch auflösenden Streuröntgenbilds $D_{tSh}$ vom hoch auflösenden Bild $D_{fh}$,

wobei das Bild $D_{fPh}$ ein hoch auflösendes zweidimensionales Bild des Subjekts (12) der Hauptröntgenstrahlen (32) ist, nachdem man die Streuröntgenstrahlen (34) im Wesentlichen beseitigt hat, und das Bild $D_{fPh}$ eine Auflösung hat, die im Wesentlichen gleich der höchsten mit der Detektorbaugruppe verfügbaren Auflösung ist.

**9.** Verfahren nach Anspruch 8, worin:

a) die Strahlauswahlvorrichtung (18) beweglich ist zwischen einer Blockierposition, in der es im Wesentlichen nur den Häuptröntgenstrahlen (32) möglich ist, die ausgewählten Erfassungsstellen zu erreichen, und einer Durchlassposition, in der es den Hauptröntgenstrahlen (32) und den Streuröntgenstrahlen (34) möglich ist, die ausgewählten Erfassungsstellen zu erreichen;
b) die Strahlauswahlvorrichtung (18) vor dem Erfassen des hoch auflösenden Rohbilds $I_{fh}$ in die Durchlassposition bewegt wird; und

c) die Strahlauswahlvorrichtung (18) vor dem Erfassen des niedrig auflösenden Rohbilds $I_{rl}$ in die Blockierposition bewegt wird.

**10.** Verfahren nach Anspruch 8, worin die Röntgenstrahlen-Detektorbaugruppe eine vordere zweidimensionale Röntgenstrahlen-Detektorbaugruppe (16) und eine rückwärtige zweidimensionale Röntgenstrahlen-Detektorbaugruppe (26) umfasst und die Strahlauswahlvorrichtung (18) dazwischen angeordnet ist, und das hoch auflösende Bild $I_{fh}$ mit den vorderen Erfassungsstellen auf der vorderen Detektorbaugruppe (16) gewonnen wird, und die gewählten Erfassungsstellen gewählte rückwärtige Erfassungsstellen auf der rückwärtigen Detektorbaugruppe (26) sind, die im Wesentlichen nur die Hauptröntgenstrahlen (32) empfangen, die die Strahlauswahlvorrichtung (18) durchlässt, und das niedrig auflösende Bild $D_{fl}$ von vorderen Erfassungsstellen erzeugt wird, die von Röntgenstrahlen-Projektionslinien geschnitten werden, die von der Röntgenstrahlenquelle (14) zu den gewähltem rückwärtigen Erfassungsstellen verlaufen.

**11.** Verfahren nach Anspruch 10, worin:

a) die Röntgenstrahlenquelle (14) dafür eingerichtet ist, Röntgenstrahlen (30) mit einem mittleren Energieniveau L zur Zeit $t_L$ und mit einem mittleren Energieniveau H zur Zeit $t_H$ auszusenden;
b) das hoch auflösende Rohbild $I_{fh}$ aus dem Niedrigenergiebild $I_{fLh}$ besteht, das von den vorderen Erfassungsstellen zur Zeit $t_L$ erfasst wird, und einem Hochenergiebild $I_{fHh}$, das von den vorderen Erfassungsstellen zur Zeit $t_H$ erfasst wird, und das Bild $D_{fh}$ aus einem Niedrigenergiebild $D_{fLh}$ besteht, das durch Verarbeiten des Niedrigenergie-Rohbilds $I_{fLh}$ berechnet wird, um es zu normieren und dunkle Signale zu subtrahieren, und aus einem Hochenergiebild $D_{fHh}$, das durch Verarbeiten des Hochenergie-Rohbilds $I_{fHh}$ berechnet wird, um es zu normieren und dunkle Signale zu subtrahieren;
c) das niedrig auflösende Bild $D_{fl}$ aus einem niedrig auflösenden Bild $D_{fLl}$ besteht, das zu den gewählten rückwärtigen Erfassungsstellen gehört und aus dem Bild $D_{fLh}$ erzeugt wird, und einem niedrig auflösenden Bild $D_{fHl}$, das zu den gewählten rückwärtigen Erfassungsstellen gehört und aus dem Bild $D_{fHh}$ erzeugt wird;
d) das niedrig auflösende Rohbild $I_{rl}$ aus einem Niedrigenergiebild $I_{rLl}$ besteht, das von den gewählten rückwärtigen Erfassungsstellen zur Zeit $t_L$ gewonnen wird, und einem Hochenergiebild $I_{rHl}$, das von den gewählten rückwärtigen Erfassungsstellen zur Zeit $t_H$ gewonnen wird, und das Bild $D_{rl}$ aus einem Niedrigenergiebild $D_{rLl}$ besteht, das durch Verarbeiten des Bilds $I_{rLl}$ berechnet wird, um es zu normieren und dunkle Signale zu subtrahieren, und einem Hochenergiebild $D_{rHl}$, das durch Verarbeiten des Bilds $I_{rHl}$ berechnet wird, um es zu normieren und dunkle Signale zu subtrahieren;
e) das Hauptröntgenstrahlenbild $D_{fPl}$ aus einem Bild $D_{fLPl}$ besteht, das durch Multiplizieren des Bilds $D_{rLl}$ mit einem vorbestimmten Niedrigenergiefaktor berechnet wird, und einem Bild $D_{fHPl}$, das durch Multiplizieren des Bilds $D_{rHl}$ mit einem vorbestimmten Hochenergiefaktor berechnet wird;
f) das niedrig auflösende Streuröntgenstrahlenbild $D_{fSl}$ aus einem Bild $D_{fLSl}$ besteht, das durch Subtrahieren des Bilds $D_{fLPl}$ vom Bild $D_{fLl}$ berechnet wird, und einem Bild $D_{fHSl}$, das durch Subtrahieren des Bilds $D_{fHPl}$ vom Bild $D_{fHl}$ berechnet wird;
g) das hoch auflösende Streuröntgenstrahlenbild $D_{fSh}$ aus einem hoch auflösenden Streuröntgenstrahlenbild $D_{fLSh}$ besteht, das durch Interpolation aus dem niedrig auflösenden Streuröntgenstrahlenbild $D_{fLSl}$ berechnet wird, und einem hoch auflösenden Streuröntgenstrahlenbild $D_{fHSh}$, das durch Interpolation aus dem niedrig auflösenden Streuröntgenstrahlenbild $D_{fHSl}$ berechnet wird; und
h) das hoch auflösende Hauptröntgenstrahlenbild $D_{fPh}$ aus einem hoch auflösenden Hauptröntgenstrahlenbild $D_{fLPh}$ besteht, das durch Subtrahieren des Bilds $D_{fLSh}$ vom Bild $D_{fLh}$ berechnet wird, und einem hoch auflösenden Hauptröntgenstrahlenbild $D_{fHPh}$, das durch Subtrahieren des Bilds $D_{fHSh}$ vom Bild $D_{fHh}$ berechnet wird,

wobei die Bilder $D_{fLPh}$ und $D_{fHPh}$ ein hoch auflösendes zweidimensionales Doppelenergie-Hauptröntgenstrahlen-Bildpaar $D_{fLPh}$, $D_{fHPh}$ des Subjekts (12) bilden, nachdem die Streuröntgenstrahlen (34) im Wesentlichen beseitigt sind, und das Bildpaar eine Auflösung hat, die im Wesentlichen gleich der höchsten Auflösung ist, die die vordere Detektorbaugruppe (16) liefern kann.

**12.** Verfahren nach Anspruch 10, worin:

a) die Röntgenstrahlenquelle (14) so ausgelegt ist, dass sie gleichzeitig Röntgenstrahlen (30) mit einem mittleren Energieniveau L und mit einem mittleren Energieniveau H aussendet, und die rückwärtige Detektorbaugruppe (26) in körperlicher Abfolge einen rückwärtigen Niederenergie-Röntgenstrahlendetektor (40), ein rückwärtiges Röntgenstrahlenenergie-Spektralfilter (42) und einen rückwärtigen Hochenergie-Röntgenstrahlendetektor (44) umfasst, und die gewählten rückwärtigen Erfassungsstellen ausgewählte rückwärtige Niederenergie-Erfassungsstellen auf dem rückwärtigen Niederenergiedetektor (40) enthalten sowie entsprechende rückwärtige Hochenergie-Erfassungsstellen auf dem rückwärtigen Hochenergiedetektor (44);

b) das niedrig auflösende Rohbild $I_{rl}$ aus einem Niederenergiebild $I_{rLl}$ besteht, das von den gewählten rückwärtigen Niederenergie-Erfassungsstellen gewonnen wird, und einem Hochenergiebild $I_{rHl}$, das von den gewählten rückwärtigen Hochenergie-Erfassungsstellen gewonnen wird, und das Bild $D_{rl}$ aus einem Miederenergiebild $D_{rLl}$ besteht, das durch Verarbeiten des Bilds $I_{rLl}$ berechnet wird, um es zu normieren und dunkle Signale zu subtrahieren, und einem Hochenergiebild $D_{rHl}$, das durch Verarbeiten des Bilds $I_{rHl}$ berechnet wird, um es zu normieren und dunkle Signale zu subtrahieren; und

c) das niedrig auflösende Hauptröntgenstrahlenbild $D_{fPl}$ aus den Bildern $D_{rLl}$ und $D_{rHl}$ berechnet wird.

**13.** Verfahren nach Anspruch 12, worin:

a) die vordere Detektorbaugruppe (16) in körperlicher Abfolge einen vorderen Niederenergie-Röntgenstrahlendetektor (50), ein vorderes Röntgenstrahlenenergie-Spektralfilter (52) und einen vorderen Hochenergie-Röntgenstrahlendetektor (54) umfasst;

b) das hoch auflösende Rohbild $I_{fh}$ aus einem Niederenergiebild $I_{fLh}$ besteht, das von den vorderen Niederenergie-Erfassungsstellen gewonnen wird, und einem Hochenergiebild $I_{fHh}$, das von den vorderen Hochenergie-Erfassungsstellen gewonnen wird, und das hoch auflösende Bild $D_{fh}$ aus einem Niederenergiebild $D_{fLh}$ besteht, das durch Verarbeiten des Bilds $I_{fLh}$ berechnet wird, um es zu normieren und dunkle Signale zu entfernen, und einem Hochenergiebild $D_{fHh}$, das durch Verarbeiten des Bilds $I_{fHh}$ berechnet wird, um es zu normieren und dunkle Signale zu entfernen;

c) das niedrig auflösende Bild $D_{fl}$ aus einem niedrig auflösenden Bild $D_{fLl}$ besteht, das zu den gewählten rückwärtigen Niederenergie-Erfassungsstellen gehört und aus dem Niederenergiebild $D_{fLh}$ erzeugt wird, und einem niedrig auflösenden Bild $D_{fHl}$, das zu den gewählten rückwärtigen Hochenergie-Erfassungsstellen gehört und aus dem Hochenergiebild $D_{fHh}$ erzeugt wird;

d) das Hauptröntgenstrahlenbild $D_{fPl}$ aus einem Bild $D_{fLPl}$ besteht, das durch Multiplizieren des Niederenergiebilds $D_{rLl}$ mit einem vorbestimmten Niederenergiefaktor berechnet wird, und einem Bild $D_{fHPl}$, das durch Multiplizieren des Hochenergiebilds $D_{rHl}$ mit einem vorbestimmten Hochenergiefaktor berechnet wird;

e) das niedrig auflösende Streuröntgenstrahlenbild $D_{fSl}$ aus einem Bild $D_{fLSl}$ besteht, das durch Subtrahieren des multiplizierten Niederenergiebilds $D_{fLPl}$ von dem niedrig auflösenden Niederenergiebild $D_{fLl}$ berechnet wird, und einem Bild $D_{fHSl}$, das durch Subtrahieren des multiplizierten Hochenergiebilds $D_{fHPl}$ von dem niedrig auflösenden Hochenergiebild $D_{fHl}$ berechnet wird;

f) das hoch auflösende Streuröntgenstrahlenbild $D_{fSh}$ aus einem hoch auflösenden Streuröntgenstrahlenbild $D_{fLSh}$ besteht, das durch Interpolation aus dem niedrig auflösenden Streuröntgenstrahlenbild $D_{fLSl}$ berechnet wird, und einem hoch auflösenden Streuröntgenstrahlenbild $D_{fHSh}$, das durch Interpolation aus dem niedrig auflösenden Streuröntgenstrahlenbild $D_{fHSl}$ berechnet wird; und

g) das hoch auflösende Hauptröntgenstrahlenbild $D_{fPh}$ aus einem hoch auflösenden Hauptröntgenstrahlenbild $D_{fLPh}$ besteht, das durch Subtrahieren des Niederenergie-Streubilds $D_{fLSh}$ von Niederenergiebild $D_{fLh}$ berechnet wird, und einem hoch auflösenden Hauptröntgenstrahlenbild $D_{fHPh}$, das durch Subtrahieren des Hochenergie-Streubilds $D_{fHSh}$ von Hochenergiebild $D_{fHh}$ berechnet wird,

wobei die Bilder $D_{fLPh}$ und $D_{fHPh}$ ein hoch auflösendes zweidimensionales Doppelenergie-Hauptröntgenstrahlen-Bildpaar $D_{fLPh}$, $D_{fHPh}$ des Subjekts (12) bilden, nachdem die Streuröntgenstrahlen (34) im Wesentlichen beseitigt sind, und das Bildpaar eine Auflösung hat, die im Wesentlichen gleich der höchsten Auflösung ist, die die vordere Detektorbaugruppe (16) liefern kann.

## Revendications

**1.** Système d'imagerie bidimensionnelle par rayons X pour prendre des images d'un sujet (12), ledit système comprenant :

(a) une source de rayons X (14) adaptée pour émettre des rayons X (30) destinés à traverser ledit sujet (12), lesdits rayons X (30) comprenant des rayons X primaires (32) dont la direction de déplacement n'est pas modifiée par l'interaction avec ledit sujet (12) et lesdits rayons X (30) comprenant des rayons X de diffusion (34) dont la direction de déplacement est modifiée par l'interaction avec ledit sujet (12) ;

(b) un moyen de détection de rayons X pour détecter lesdits rayons X en deux dimensions,

(c) un moyen de sélection (18) de faisceau pour bloquer le passage de substantiellement tous lesdits rayons X de diffusion (34) et permettre le passage d'une partie desdits rayons X primaires (32) ;

dans lequel ledit sujet (12) est situé entre ladite source de rayons X (14) et ledit moyen de détection de rayons X ; et

ledit moyen de détection comprend un moyen de détection à haute résolution pour détecter lesdits rayons X primaires (32) et lesdits rayons X de diffusion (34) simultanément, et un moyen de détection à basse résolution pour détecter seulement lesdits rayons X (30) ayant passé ledit moyen de sélection (18) de faisceau.

**2.** Système d'imagerie par rayons X selon la revendication 1, dans lequel ledit moyen de sélection (18) de faisceau est substantiellement composé d'un matériau absorbant les rayons X comportant une pluralité de trous (20), les axes desdits trous (20) étant parallèles à la direction de déplacement desdits rayons X primaires (32).

**3.** Système d'imagerie par rayons X selon la revendication 1 ou 2, dans lequel ledit moyen de détection comprend un ensemble formant détecteur (16) et dans lequel ledit moyen de sélection (18) de faisceau a deux positions, une position transmissive dans laquelle ledit ensemble formant détecteur agit en tant que ledit moyen de détection à haute résolution, et une position de blocage dans laquelle ledit ensemble formant détecteur agit en tant que ledit moyen de détection à basse résolution.

**4.** Système d'imagerie par rayons X selon la revendication 1 ou 2, dans lequel ledit moyen de détection à haute résolution comprend un ensemble formant détecteur avant (16) et ledit moyen de détection à basse résolution comprend un ensemble formant détecteur arrière (26), et dans lequel ledit système comprend, en séquence physique de l'avant vers l'arrière, ladite source de rayons X (14), ledit ensemble formant détecteur avant (16), ledit moyen de sélection (18) de faisceau et ledit ensemble formant détecteur arrière (26), ledit sujet (12) étant situé entre ladite source de rayons X (14) et ledit ensemble formant détecteur avant (16).

**5.** Système d'imagerie par rayons X selon l'une quelconque des revendications précédentes, dans lequel ladite source de rayons X (14) est adaptée pour émettre des impulsions de rayons X avec deux énergies moyennes différentes.

**6.** Système d'imagerie par rayons X selon les revendications 4 et 5, dans lequel ledit ensemble formant détecteur arrière (26) comprend, en séquence physique de l'avant vers l'arrière, un détecteur basse énergie arrière (40), un filtre spectral d'énergie de rayons X arrière (42), et un détecteur haute énergie arrière (44), ledit détecteur basse énergie arrière (40) comportant une pluralité de cellules détectrices sensibles aux rayons X agencées en matrice sensiblement rectangulaire, ledit détecteur haute énergie arrière (44) comportant une pluralité de cellules détectrices sensibles aux rayons X, l'agencement et la quantité desdites cellules détectrices haute énergie arrière étant sensiblement les mêmes que l'agencement et la quantité desdites cellules détectrices basse énergie arrière.

**7.** Système d'imagerie par rayons X selon la revendication 6, dans lequel ledit ensemble formant détecteur (16) comporte, en séquence physique de l'avant vers l'arrière, un détecteur basse énergie avant (50), un filtre spectral d'énergie de rayons X avant (52), et un détecteur haute énergie avant (54), ledit détecteur basse énergie avant (50) comportant une pluralité de cellules détectrices sensibles aux rayons X agencées en matrice sensiblement rectangulaire, ledit détecteur haute énergie avant (54) comportant une pluralité de cellules détectrices sensibles aux rayons X, l'agencement et la quantité desdites cellules détectrices haute énergie avant étant sensiblement les mêmes que l'agencement et la quantité desdites cellules détectrices basse énergie avant.

**8.** Procédé pour prendre une image bidimensionnelle par rayons X d'un sujet (12) en utilisant un système d'imagerie bidimensionnelle par rayons X, ledit sujet (12) étant composé substantiellement de deux matériaux qui interagissent différemment avec des rayons X (30), ledit système d'imagerie comportant une source de rayons X (14), un moyen de sélection (18) de faisceau et un ensemble formant détecteur de rayons X bidimensionnel ayant une pluralité d'emplacements de détection, ledit sujet (12) étant entre ladite source de rayons X (14) et ledit ensemble formant détecteur, ladite source de rayons X (14) étant adaptée pour émettre des rayons X (30) destinés à traverser ledit sujet (12), comprenant des rayons X primaires (32) dont la direction de déplacement n'est pas modifiée par l'interaction avec ledit sujet (12), et des rayons X de diffusion (34) dont la direction de déplacement est modifiée par l'interaction avec ledit sujet (12), ledit moyen de sélection (18) de faisceau permettant sélectivement et sensiblement uniquement aux rayons X primaires (32) d'atteindre des emplacements de détection choisis, ledit procédé comprenant les opérations consistant à :

(a) éclairer ledit sujet (12) avec lesdits rayons X (30) ;

(b) acquérir une image haute résolution brute $I_{fh}$ à partir desdits emplacements de détection et traiter ladite image $I_{fh}$ pour la normaliser et pour soustraire les signaux sombres, pour obtenir une image haute résolution $D_{fh}$ composée desdits rayons X primaires (32) et desdits rayons X de diffusion (34) ;

(c) produire, à partir de ladite image $D_{fh}$, une image basse résolution $D_{fl}$ correspondant auxdits emplacements de détection choisis ;

(d) acquérir une image basse résolution brute $I_{rl}$ à partir desdits emplacements de détection choisis et traiter ladite image $I_{rl}$ pour la normaliser et pour soustraire les signaux sombres, pour obtenir une image basse résolution $D_{rl}$ composée sensiblement uniquement desdits rayons X primaires (32) ;

(e) calculer une image de rayons X primaires basse résolution $D_{fPl}$ correspondant auxdits emplacements de détection choisis en multipliant ladite image $D_{rl}$ par un facteur prédéfini qui est indépendant dudit sujet (12) ;

(f) calculer une image de rayons X de diffusion basse résolution $D_{fSl}$ correspondant auxdits emplacements de détection choisis en soustrayant ladite image $D_{fPl}$ de ladite image $D_{fl}$ ;

(g) calculer une image de rayons X de diffusion haute résolution $D_{fSh}$ par interpolation à partir de ladite image de rayons X de diffusion basse résolution $D_{fSl}$ ; et

(h) calculer une image de rayons X primaires haute résolution $D_{fPh}$ en soustrayant ladite image de rayons X de diffusion haute résolution $D_{fSh}$ de ladite image haute résolution $D_{fh}$ ;

grâce à quoi ladite image $D_{fPh}$ est une image de rayons X primaires (32) bidimensionnelle à haute résolution dudit sujet (12) après que lesdits rayons X de diffusion (34) ont été sensiblement éliminés, ladite image $D_{fPh}$ ayant une résolution sensiblement égale à la plus haute résolution pouvant être obtenue avec ledit ensemble formant détecteur.

9. Procédé selon la revendication 8, dans lequel :

(a) ledit moyen de sélection (18) de faisceau est mobile entre une position de blocage qui permet sensiblement uniquement aux rayons X primaires (32) d'atteindre lesdits emplacements de détection choisis et une position transmissive qui permet à la fois auxdits rayons x primaires (32) et auxdits rayons X de diffusion (34) d'atteindre lesdits emplacements de détection choisis ;

(b) ledit moyen de sélection (18) de faisceau est déplacé jusqu'à ladite position transmissive avant l'acquisition de ladite image haute résolution brute $I_{fh}$ ; et

(c) ledit moyen de sélection (18) de faisceau est déplacé jusqu'à ladite position de blocage avant l'acquisition de ladite image basse résolution brute $I_{rl}$.

10. Procédé selon la revendication 8, dans lequel ledit ensemble formant détecteur de rayons X comprend un ensemble formant détecteur de rayons X bidimensionnel avant (16) et un ensemble formant détecteur de rayons X bidimensionnel arrière (26), ledit moyen de sélection (18) de faisceau étant positionné entre eux, ladite image haute résolution $I_{fh}$ étant acquise à partir d'emplacements de détection avant sur ledit ensemble formant détecteur avant (16), et lesdits emplacements de détection choisis sont des emplacements de détection arrière choisis sur ledit ensemble formant détecteur arrière (26) recevant sensiblement uniquement lesdits rayons X primaires (32) qui sont passés par ledit moyen de sélection (18) de faisceau, ladite image basse résolution $D_{fl}$ étant produite à partir d'emplacements de détection avant coupés par des droites de projection de rayons X s'étendant de ladite source de rayons X (14) auxdits emplacements de détection arrière.

11. Procédé selon la revendication 10, dans lequel :

(a) ladite source de rayons X (14) est adaptée pour émettre des rayons X (30) à un niveau d'énergie moyen L à un instant $t_L$ et à un niveau d'énergie moyen H à un instant $t_H$ ;

(b) ladite image haute résolution brute $I_{fh}$ est constituée d'une image basse énergie $I_{fLh}$ acquise à partir desdits emplacements de détection avant à l'instant $t_L$ et d'une image haute énergie $I_{fHh}$ acquise à partir desdits emplacements de détection avant à l'instant $t_H$, et ladite image $D_{fh}$ est constituée d'une image basse énergie $D_{fLh}$ calculée en traitant ladite image basse énergie brute $I_{fLh}$ pour la normaliser et pour soustraire les signaux sombres, et d'une image haute énergie $D_{fHh}$ calculée en traitant ladite image haute énergie brute $I_{fHh}$ pour la normaliser et pour soustraire les signaux sombres ;

(c) ladite image basse résolution $D_{fl}$ est constituée d'une image basse résolution $D_{fLl}$ correspondant auxdits emplacements de détection arrière choisis et produite à partir de ladite image $D_{fLh}$, et d'une image basse résolution $D_{fHl}$ correspondant auxdits emplacements de détection arrière choisis et produite à partir de ladite image $D_{fHh}$ ;

(d) ladite image basse résolution brute $I_{rl}$ est constituée d'une image basse énergie $I_{rL1}$ acquise à partir desdits emplacements de détection arrière choisis à l'instant $t_L$ et d'une image haute énergie $I_{rHl}$ acquise à partir desdits emplacements de détection arrière choisis à l'instant $t_H$, et ladite image $D_{rl}$ est constituée d'une image basse énergie $D_{rLl}$ calculée en traitant ladite image $I_{rLl}$ pour la normaliser et pour soustraire les signaux sombres, et d'une image haute énergie $D_{rHl}$ calculée en traitant ladite image $I_{rHl}$ pour la normaliser et pour soustraire les signaux sombres ;

(e) ladite image de rayons X primaires $D_{fPl}$ est constituée d'une image $D_{fLPl}$ calculée en multipliant ladite image $D_{rLl}$ par un facteur de basse énergie prédéfini, et d'une image $D_{fHPl}$ calculée en multipliant ladite image $D_{rHl}$ par un facteur de haute énergie prédéfini ;

(f) ladite image de rayons X de diffusion basse résolution $D_{fSl}$ est constituée d'une image $D_{fLSl}$ calculée en soustrayant ladite image $D_{fLPl}$ de ladite image $D_{fLl}$, et d'une image $D_{fHSl}$ calculée en soustrayant ladite image $D_{fHPl}$ de ladite image $D_{fHl}$ ;

(g) ladite image de rayons X de diffusion haute résolution $D_{fSh}$ est constituée d'une image de rayons X de diffusion haute résolution $D_{fLSh}$ calculée par interpolation à partir de ladite image de rayons X de diffusion basse résolution $D_{fLSl}$, et d'une image de rayons X de diffusion haute résolution $D_{fHSh}$ calculée par interpolation à partir de ladite image de rayons X de diffusion basse résolution $D_{fHSl}$ ; et

(h) ladite image de rayons X primaires haute résolution $D_{fPh}$ est constituée d'une image de rayons X primaires haute résolution $D_{fLPh}$ calculée en soustrayant ladite image $D_{fLSh}$ de ladite image $D_{fLh}$, et d'une image de rayons X primaires haute résolution $D_{fHPh}$ calculée en soustrayant ladite image ladite image $D_{fHSh}$ de ladite image $D_{fHh}$ ;

grâce à quoi lesdites images $D_{fLPh}$ et $D_{fHPh}$ forment une paire d'images de rayons X primaires haute résolution, bidimensionnelles et à double énergie $D_{fLPh}$, $D_{fHPh}$ dudit sujet (12) après que lesdits rayons X de diffusion (34) ont été substantiellement éliminés, ladite paire d'images ayant une résolution sensiblement égale à la plus haute résolution pouvant être obtenue avec ledit ensemble formant détecteur avant (16).

**12.** Procédé selon la revendication 10, dans lequel :

(a) ladite source de rayons X (14) est adaptée pour émettre des rayons X (30) à un niveau d'énergie moyen L et à un niveau d'énergie moyen H simultanément, ledit ensemble formant détecteur arrière (26) comporte, en ordre physique, un détecteur de rayons X basse énergie arrière (40), un filtre spectral d'énergie de rayons X arrière (42), et un détecteur de rayons X haute énergie arrière (44), lesdits emplacements de détection arrière choisis comprenant des emplacements de détection basse énergie arrière choisis au niveau dudit détecteur basse énergie arrière (40) et des emplacements de détection haute énergie arrière correspondants au niveau dudit détecteur haute énergie arrière (44) ;

(b) ladite image basse résolution brute $L_{rl}$ est constituée d'une image basse énergie $I_{rLl}$ acquise à partir desdits emplacements de détection basse énergie arrière choisis et d'une image haute énergie $I_{rHl}$ acquise à partir desdits emplacements de détection haute énergie arrière choisis, et ladite image $D_{rl}$ est constituée d'une image basse énergie $D_{rLl}$ calculée en traitant ladite image $I_{rLl}$ pour la normaliser et pour soustraire les signaux sombres, et d'une image haute énergie $D_{rHl}$ calculée en traitant ladite image $I_{rHl}$ pour la normaliser et pour soustraire les signaux sombres ; et

(c) ladite image de rayons X primaires basse résolution $D_{fPl}$ est calculée à partir desdites images $D_{rLl}$ et $D_{rHl}$.

**13.** Procédé selon la revendication 12, dans lequel :

(a) ledit ensemble formant détecteur avant (16) comprend, en ordre physique, un détecteur de rayons X basse énergie avant (50), un filtre spectral d'énergie de rayons X avant (52), et un détecteur de rayons X haute énergie avant (54) ;

(b) ladite image haute résolution brute $I_{fh}$ est constituée d'une image basse énergie $I_{fLh}$ acquise à partir desdits emplacements de détection basse énergie avant et d'une image haute énergie $I_{fHh}$ acquise à partir desdits emplacements de détection haute énergie avant, et ladite image haute résolution $D_{fh}$ est constituée d'une image basse énergie $D_{fLh}$ calculée en traitant ladite image $I_{fLh}$ pour la normaliser et pour soustraire les signaux sombres, et d'une image haute énergie $D_{fHh}$ calculée en traitant ladite image $I_{fHh}$ pour la normaliser et pour soustraire les signaux sombres ;

(c) ladite image basse résolution $D_{fl}$ est constituée d'une image basse résolution $D_{fLl}$ correspondant auxdits emplacements de détection basse énergie arrière choisis et produite à partir de ladite image basse énergie $D_{fLh}$, et d'une image basse résolution $D_{fHl}$ correspondant aux emplacements de détection haute énergie arrière choisis et produite à partir de ladite image haute énergie $D_{fHh}$ ;

(d) ladite image de rayons X primaires $D_{fPl}$ est constituée d'une image $D_{fLPl}$ calculée en multipliant ladite image basse énergie $D_{rLl}$ par un facteur basse énergie prédéfini, et d'une image $D_{fHPl}$ calculée en multipliant ladite image haute énergie $D_{rHl}$ par un facteur haute énergie prédéfini ;

(e) ladite image de rayons X de diffusion basse résolution $D_{fSl}$ est constituée d'une image $D_{fLSl}$ calculée en soustrayant ladite image basse énergie multipliée $D_{fLPl}$ de ladite image basse résolution basse énergie $D_{fLl}$, et d'une image $D_{fHSl}$ calculée en soustrayant ladite image haute énergie multipliée $D_{fHPl}$ de ladite image basse résolution haute énergie $D_{fHl}$ ;

(f) ladite image de rayons x de diffusion haute résolution $D_{fSh}$ est constituée d'une image de rayons X de diffusion haute résolution $D_{fLSh}$ calculée par interpolation à partir de ladite image de rayons X de diffusion basse résolution $D_{fLSl}$, et d'une image de rayons X de diffusion haute résolution $D_{fHSh}$ calculée par interpolation à partir de ladite image de rayons X de diffusion basse résolution $D_{fHSl}$ ; et

(g) ladite image de rayons X primaires haute résolution $D_{fPh}$ est constituée d'une image de rayons X primaires haute résolution $D_{fLPh}$ calculée en soustrayant ladite image de diffusion basse énergie $D_{fLSh}$ à partir de ladite image basse énergie $D_{fLh}$, et une image de rayons X primaires haute résolution $D_{fHPh}$ calculée en soustrayant ladite image de diffusion haute énergie $D_{fHSh}$ de ladite image haute énergie $D_{fHh}$ ;

grâce à quoi lesdites images $D_{fLPh}$ et $D_{fHPh}$ forment une paire d'images de rayons X primaires haute résolution, bidimensionnelles et à double énergie $D_{fLPh}$, $D_{fHPh}$ dudit sujet (12) après que lesdits rayons X de diffusion (34) ont été substantiellement éliminés, ladite paire d'images ayant une résolution sensiblement égale à la plus haute résolution pouvant être obtenue avec ledit ensemble formant détecteur avant (16).

FIG. 1

FIG. 2

Normalization of detector cells in order to use a single detector cell to represent all detector cells

Calibration with experimental data

Maintain an accuracy as high as using real number analytical calculations can provide at each step

For any data pair (b,s), find $(D_H, D_L)$

Constructing a nonlinear equation system $D_H = D_H(b,s)$ and $D_L = D_H(b,s)$

Numerical inversion of the nonlinear equation system for $D_H$ and $D_L$

For any data pair $(D_H, D_L)$, find (b,s)

Reconstructing an equation system $b = b(D_H, D_L)$ and $s = s(D_H, D_L)$

FIG. 3

24

**FIG. 4**

Illuminate subject with x-rays

↓

Retrieve $I_{rHI}$ and $I_{rLI}$ from rear detector and process to normalize and subtract dark signals, yielding image pair $D_{rHI}$ and $D_{rLI}$

↓

Solve image pair $D_{rHI}$ and $D_{rLI}$ to determine subject materials b and s

↓

Retrieve image $I_{fh}$ from front detector and process to normalize and subtract dark signals, yielding image $D_{fh}$

↓

Determine scatter component $D_{fSI}$ of $D_{fh}$ at detector cells on the projection lines by inserting values for b and s and solving for $D_{fSI}$

↓

Interpolate $D_{fSI}$ for front detector cells not on projection lines, yielding scatter image $D_{fSh}$

↓

Subtract $D_{fSh}$ from $D_{fh}$ to yield image $D_{fPh}$

**FIG. 6**

Illuminate subject with x-rays

↓

Retrieve image $D_{fh}(x,y)$ from front detector

↓

Retrieve $D_{rHI}(i,j)$ and $D_{rLI}(i,j)$ from rear detector

↓

Solve image pair $D_{rHI}(i,j)$ and $D_{rLI}(i,j)$ to determine subject materials b and s

↓

Determine scatter-free image $D_{fPI}(x(i),y(j))$ by inserting values for b and s

↓

Determine scatter image $D_{fSI}(x(i),y(j))$ by subtracting scatter-free image $D_{fPI}(x(i),y(j))$ from portion of $D_{fh}$ on projection lines

↓

Interpolate DfSI(x(i),y(j)) for front detector cells not on projection lines, yielding scatter image $D_{fSh}(x,y)$

↓

Subtract $D_{fSh}(x,y)$ from $D_{fh}(x,y)$ to yield image $D_{fPh}(x,y)$

**FIG. 5**

**FIG. 7a**

FIG. 7b

## FIG. 8 Flowchart

Illuminate subject with x-rays

↓

Move beam selector means away from subject and detector

↓

Retrieve image $D_{th}(x,y)$ from detector

↓

Move beam selector means between subject and detector

↓

Retrieve image $D_{rl}(i,j)$ from detector

↓

Determine the low-resolution primary image $D_{lPl}(x(i),y(j)) = D_{rl}(i,j) \times C_g(i,j)$, where $C_g(i,j)$ is a predetermined constant independent of the image subject

↓

Determine the low-resolution scatter image $D_{lSl}(x(i),y(j))$ by subtracting scatter-free image $D_{lPl}(x(i),y(j))$ from $D_{th}(x(i),y(j))$

↓

Interpolate $D_{lSl}(x(i),y(j))$ for front detector cells not on projection lines, yielding scatter image $D_{lSh}(x,y)$

↓

Subtract $D_{lSh}(x,y)$ from $D_{th}(x,y)$ to yield image $D_{lPh}(x,y)$

## FIG. 8

## FIG. 10 Flowchart

Illuminate subject with x-rays of energy H

↓

Retrieve image $D_{fHh}(x,y)$ from front detector

↓

Retrieve $D_{rHl}(i,j)$ from rear detector

↓

Illuminate subject with x-rays of energy L

↓

Retrieve image $D_{fLh}(x,y)$ from front detector

↓

Retrieve $D_{rLl}(i,j)$ from rear detector

↓

Solve image pair $D_{rHl}(i,j)$ and $D_{rLl}(i,j)$ to determine subject materials b and s

↓

Determine scatter-free image pair $D_{fLPl}(x(i),y(j))$ and $D_{fHPl}(x(i),y(j))$ by inserting values for b and s

↓

Determine scatter image $D_{fHSl}(x(i),y(j))$ by subtracting scatter-free images $D_{fHPl}(x(i),y(j))$ from portion of $D_{fHh}$ on projection lines and determine scatter image $D_{fLSl}(x(i),y(j))$ by subtracting scatter-free images $D_{fLPl}(x(i),y(j))$ from portions of $D_{fLh}$ on projection lines

↓

Interpolate $D_{fHSl}(x(i),y(j))$ for front detector cells not on projection lines, yielding scatter image $D_{fHSh}(x,y)$, and interpolate $D_{fLSl}(x(i),y(j))$ for front detector cells not on projection lines, yielding scatter image $D_{fLSh}(x,y)$

↓

Subtract $D_{fHSh}(x,y)$ from $D_{fHh}(x,y)$ to yield image $D_{fHPh}(x,y)$ and Subtract $D_{fLSh}(x,y)$ from $D_{fLh}(x,y)$ to yield image $D_{fLPh}(x,y)$

## FIG. 10

FIG. 9

FIG. 11

```
┌─────────────────────────────────────────┐
│        Illuminate subject with x-rays     │
└─────────────────────────────────────────┘
                    │
                    ▼
┌─────────────────────────────────────────┐
│   Retrieve images D_{fHh}(x,y) and D_{fLh}(x,y) │
│            from front detector            │
└─────────────────────────────────────────┘
                    │
                    ▼
┌─────────────────────────────────────────┐
│   Retrieve images D_{rHl}(i,j) and D_{rLl}(i,j)  │
│             from rear detector            │
└─────────────────────────────────────────┘
                    │
                    ▼
┌─────────────────────────────────────────┐
│  Solve image pair D_{rHl}(i,j) and D_{rLl}(i,j) to │
│     determine subject materials b and s    │
└─────────────────────────────────────────┘
                    │
                    ▼
┌─────────────────────────────────────────┐
│         Determine scatter-free image      │
│  pair D_{fLPl}(x(i),y(j)) and D_{fHPl}(x(i),y(j)) by │
│         inserting values for b and s      │
└─────────────────────────────────────────┘
                    │
                    ▼
┌─────────────────────────────────────────┐
│  Determine scatter image D_{fHSl}(x(i),y(j)) by │
│ subtracting scatter-free images D_{fHPl}(x(i),y(j)) │
│   from portion of D_{fHh} on projection lines and │
│    determine scatter image D_{fLSl}(x(i),y(j)) by │
│ subtracting scatter-free images D_{fLPl}(x(i),y(j)) │
│     from portions of D_{fLh} on projection lines │
└─────────────────────────────────────────┘
                    │
                    ▼
┌─────────────────────────────────────────┐
│  Interpolate D_{fHSl}(x(i),y(j)) for front detector │
│      cells not on projection lines, yielding │
│    scatter image D_{fHSh}(x,y), and interpolate │
│    D_{fLSl}(x(i),y(j)) for front detector cells not │
│          on projection lines, yielding    │
│          scatter image D_{fLSh}(x,y)      │
└─────────────────────────────────────────┘
                    │
                    ▼
┌─────────────────────────────────────────┐
│   Subtract D_{fHSh}(x,y) from D_{fHh}(x,y) to yield │
│   image D_{fHPh}(x,y) and subtract D_{fLSh}(x,y) from │
│     D_{fLh}(x,y) to yield image D_{fLPh}(x,y)  │
└─────────────────────────────────────────┘
```

## FIG. 12

FIG. 13a

FIG. 13b

FIG. 13c

FIG. 13d